# EUROPEAN PATENT APPLICATION

(11) **EP 1 862 133 A1**
(43) Date of publication of application: **05.12.2007**
(21) Application number: 06011489.9
(22) Date of filing: 02.06.2006
(51) Int. Cl.: A61B 17/32

(54) **Ultrasonic surgical apparatus and method of driving ultrasonic treatment device**

(71) Applicant: OLYMPUS MEDICAL SYSTEMS CORP., Tokyo (JP)
(72) Inventor: Tanaka, Kazue, Kanagawa-ken 229-0038 (JP); Konishi, Sumihito, Tokyo 181-0011 (JP); Kubota, Tatsuya, Yokohama 227-0038 (JP); Sakurai, Tomohisa, Kanagawa-ken 229-1124 (JP); Hatta, Shinji, Tokyo 192-0913 (JP); Ono, Hiroo, Tokyo 184-0003 (JP); Baltes, Hubert, 20041 Hamburg (JP)
(74) Representative: Schaefer, Konrad

(57) **Abstract**

An ultrasonic surgical apparatus comprises an ultrasonic transducer for generating vibration in response to a drive signal to be given, and a treatment device having a probe to which the vibration is transferred from the ultrasonic transducer to effect treatment with the vibration. This device further comprises a signal generator for generating an AC signal for driving the ultrasonic transducer, and a modulator for modulating the AC signal generated by the signal generator to produce a drive signal to be given to the ultrasonic transducer.

## Description

### CROSS REFERENCES TO RELATED APPLICATION

The present application relates to and incorporates by reference Japanese Patent application No. 2004-351801 filed on December 3, 2004.

### Background of the Invention

### (Field of the Invention)

The present invention relates to an ultrasonic surgical apparatus and a method for driving an ultrasonic treatment device, and in particular, to an ultrasonic surgical apparatus for effecting treatment by holding living tissue which is subjected to surgery, and a method for driving an ultrasonic treatment device.

### (Description of Related Art)

Ultrasonic surgical apparatuses have been in practical use, by which living tissue is incised by allowing a treatment device to vibrate with ultrasonic mechanical vibration.
An example of such an ultrasonic surgical apparatus is disclosed in Japanese Unexamined Application Publication No. H09-299381. This ultrasonic surgical apparatus has an ultrasonic treatment device which is provided with a gripper and a probe in order to grip a living tissue portion to be treated. An ultrasonic transducer is coupled to the probe to transfer longitudinal vibration of the transducer to the probe. The probe mechanically vibrates when a predetermined electrical signal is supplied to the ultrasonic transducer. When living tissue is gripped between the gripper opened/closed by a drive power and the probe to which ultrasonic vibration is transferred, the living tissue portion can be incised by the frictional heat generated between the vibrating probe and the living tissue portion.

However; magnitude (amplitude) of ultrasonic vibration generated in a probe is determined by the amplitude of current supplied to an ultrasonic transducer. An electrical signal supplied to an ultrasonic transducer has an ultrasonic frequency which is outputted while a foot switch is being turned on by an operator.

For this reason, an electrical signal has been supplied to an ultrasonic transducer to allow a probe to have constant amplitude while the foot switch is turned on. Therefore, the temperature of a treatment device has often exceeded a desired value.

### Summery of the Invention

The present invention has been made in view of the problem described above, and provides an ultrasonic surgical apparatus with improved performance for the control of the heat generation in a treatment device thereof.

One aspect of the present invention is to provide an ultrasonic surgical apparatus comprising an ultrasonic transducer for generating vibration in response to the input of a drive signal, a treatment device having a probe to which the vibration is transferred from the ultrasonic transducer, a signal generator for generating an AC (alternating current) signal for driving the ultrasonic transducer, and a modulator for modulating the AC signal generated by the signal generator to produce the drive signal and for giving the drive signal to the ultrasonic transducer.
Another aspect of the present invention is to provide a method of driving an ultrasonic treatment device provided with an ultrasonic transducer, the method comprising steps of producing an AC (alternating current) signal for driving the ultrasonic transducer, modulating amplitude of the AC signal, so that a duty ratio "T1 / (T1+T2)" is 5% to 100% and a period "T1+T2" is 0.1 seconds to 1 seconds (T1=high output period, T2=low output period) to produce a drive signal, and supplying the drive signal to the ultrasonic transducer.

### Brief Description of the Drawings

In the appended drawings:
Fig. 1 is a block diagram showing a configuration of an entire ultrasonic surgical apparatus related to a first embodiment of the present invention;
Fig. 2 is a block diagram showing a configuration of an electric circuit of the ultrasonic surgical apparatus;
Fig. 3 is a waveform diagram showing an example of a first waveform pattern applicable to the embodiment, in which amplitude of a drive current has been modulated;
Fig. 4 is a waveform diagram showing an example of a second waveform pattern applicable to the embodiment, in which amplitude of a drive current has been modulated;
Fig. 5 is a waveform diagram showing an example of a third waveform pattern applicable to the embodiment, in which amplitude of a drive current has been modulated;
Fig. 6 is a waveform diagram showing a waveform pattern data used for producing the first waveform pattern;
Fig. 7 is a waveform diagram showing a waveform pattern data used for producing the second waveform pattern;
Fig. 8 is a waveform diagram showing a waveform pattern data used for producing the third waveform pattern;
Fig. 9 is a waveform diagram showing an example of a fourth waveform pattern applicable to the embodiment, in which amplitude of a drive current has been modulated;
Fig. 10 is a waveform diagram showing a waveform pattern data used for producing the fourth waveform pattern;
Fig. 11 is a waveform diagram showing an example of a fifth waveform pattern applicable to the embodiment, in which amplitude of a drive current has been modulated;
Fig. 12 is a waveform diagram showing a waveform pattern data used for producing the fifth waveform pattern;
Fig. 13 is a waveform diagram showing an example of a sixth waveform pattern applicable to the embodiment, in which amplitude of a drive current has been modulated;
Fig. 14 is a waveform diagram showing a waveform pattern data used for producing the sixth waveform pattern;
Fig. 15 is a waveform diagram showing an example of a seventh waveform pattern applicable to the embodiment, in which amplitude of a drive current has been modulated;
Fig. 16 is a waveform diagram showing a waveform pattern data used for producing the seventh waveform pattern;
Fig. 17 is a waveform diagram showing an example of an eighth waveform pattern applicable to the embodiment, in which amplitude of a drive current has been modulated;
Fig. 18 is a waveform diagram showing a waveform pattern data used for producing the eighth waveform pattern;
Fig. 19 is a waveform diagram showing an example of a ninth waveform pattern applicable to the embodiment, in which amplitude of a drive current has been modulated;
Fig. 20 is a waveform diagram showing a waveform pattern data used for producing the ninth waveform pattern;
Fig. 21 is a waveform diagram showing an example of a tenth waveform pattern (waveform pattern data) applicable to the embodiment, in which amplitude of a drive current has been modulated;
Fig. 22 is a waveform diagram showing an example of an eleventh waveform pattern (waveform pattern data) applicable to the embodiment, in which amplitude of a drive current has been modulated;
Fig. 23 is a waveform diagram showing an example of a twelfth waveform pattern (waveform pattern data) applicable to the embodiment, in which amplitude of a drive current has been modulated;
Fig. 24 is a graph for explaining an example of temperature variation at a treatment device of a handpiece, in comparison conventional temperature variation;
Fig. 25 is a waveform diagram showing an example of a thirteenth waveform pattern (waveform pattern data) applicable to the embodiment,
   in which amplitude of a drive current has been modulated, the waveform being for simultaneously changing amplitude of a current and a duty ratio;
Fig. 26 is an electrical block diagram showing a second embodiment of the ultrasonic surgical apparatus according to the present invention;
Fig. 27 shows an example of a front panel for setting a waveform pattern data in a third embodiment of the ultrasonic surgical apparatus according to the present invention;
Fig. 28 shows an example of a front panel for setting a waveform pattern data in a fourth embodiment of the ultrasonic surgical apparatus according to the present invention;
Fig. 29 is a flow diagram showing an example of processing in a CPU for selecting a waveform pattern, which is performed in the fourth embodiment;
Fig. 30 shows an example of front panel indication in case a waveform pattern is selected in the fourth embodiment;
Fig. 31 shows an example of a fourteenth waveform pattern data;
Fig. 32 shows an example of a front panel for setting a waveform pattern data in a fifth embodiment of the ultrasonic surgical apparatus according to the present invention;
Fig. 33 is a flowchart diagram showing an example of processing in a CPU for selecting a waveform pattern, which is performed in the fifth embodiment;
Fig. 34 shows an example of a front panel indication in case a waveform pattern is selected in the fifth embodiment;
Fig. 35 is a perspective illustration of a treatment device in which an output value of a temperature sensor serves as a trigger signal, in a sixth embodiment of the ultrasonic surgical apparatus according to the present invention;
Fig. 36 is a cross section of a tip of the probe shown in Fig. 35 taken along a dotted line A;
Fig. 37 is an electrical block diagram of a main unit provided with a temperature detection circuit, in the sixth embodiment;
Fig. 38 is a perspective illustration of a treatment device in which a temperature sensor is provided to a gripper, in a seventh embodiment of the ultrasonic surgical apparatus according to the present invention;
Fig. 39 is a cross section of a tip of the probe shown in Fig. 38;
Fig. 40 shows a fifteenth waveform pattern in which a time-out signal serves as a trigger signal, in an eighth embodiment of the ultrasonic surgical apparatus according to the present invention;
Fig. 41 shows a sixteenth waveform pattern in which a time-out signal serves as a trigger signal, in a ninth embodiment of the ultrasonic surgical apparatus according to the present invention;
Fig. 42 is a flow diagram exemplifying processing in a CPU to allow a drive current modulated in amplitude according to a trigger signal in the ninth embodiment to be supplied to a handpiece;
Fig. 43 is a perspective illustration of a handpiece in which an output switch is provided at one side of an operation handle, in a tenth embodiment of the ultrasonic surgical apparatus according to the present invention;
Fig. 44 is an electrical block diagram showing a circuit configuration of a main unit provided in a switch detection circuit, in the tenth embodiment;
Fig. 45 shows a seventeenth waveform pattern data used in the tenth embodiment;
Fig. 46 is a perspective illustration of a handpiece in which an angle sensor is provided at an operation handle, in an eleventh embodiment of the ultrasonic surgical apparatus according to the present invention;
Fig. 47 is an electrical block diagram showing a circuit configuration of a main unit provided with an angle detection circuit, in the eleventh embodiment;
Fig. 48 is a perspective illustration of a handpiece in which a power sensor is provided at an operation handle, in a twelfth embodiment of the ultrasonic surgical apparatus according to the present invention;
Fig. 49 is an electrical block diagram showing a circuit configuration of a main unit provided with a power detection circuit, in the twelfth embodiment;
Fig. 50 is an electrical block diagram showing a circuit configuration of an main unit in which impedance serves as a trigger signal, in t thirteenth embodiment of the ultrasonic surgical apparatus according to the present invention;
Fig. 51 is a graph in which a duty ratio is changed relative to impedance, in a fourteenth embodiment of the ultrasonic surgical apparatus according to the present invention;
Fig. 52 shows an example of an eighteenth waveform pattern data applied to the fourteenth embodiment;
Fig. 53 shows an example of a nineteenth waveform pattern data applied to the fourteenth embodiment;
Fig. 54 is a perspective illustration for explaining a state where an RF-ID tag is provided to a handpiece at such a position on the surface thereof that can be seen when the handpiece is placed in a tray, in a fifteenth embodiment of the ultrasonic surgical apparatus according to the present invention;
Figs. 55A and 55B are characteristic diagrams of impedance and phase difference, respectively, centering on resonance frequency of an ultrasonic transducer, in a sixteenth embodiment of the ultrasonic surgical apparatus according to the present invention;
Figs. 56A to 56D show waveforms for explaining the processing of a CPU, in the sixteenth embodiment;
Fig. 57 is a graph of an experimental data showing an example of temperature variation at a treatment device, which varies relative to the variation of duty ratio;
Fig. 58 is a graph exemplifying an ideal temperature curve for temperature control performed as a modification of the sixth embodiment;
Fig. 59 is a schematic flow diagram exemplifying processes in a CPU, which is performed in a modification of the sixth embodiment; and
Fig. 60 is a graph for explaining an example of control of duty ratio according to the processing shown in Fig. 59.

### Detailed Description of Preferred Embodiments

Hereinafter, various embodiments of the ultrasonic surgical apparatus according to the present invention will now be described with reference to the accompanying drawings.

### (First embodiment)

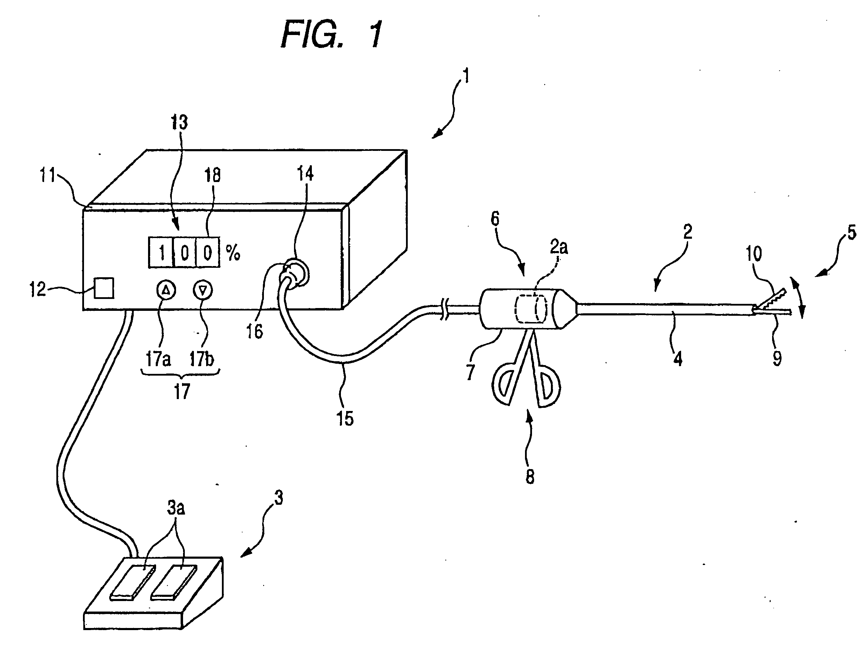

With reference to Fig. 1-25 and 57, a first embodiment of the ultrasonic surgical apparatus according to the present invention is described.
Fig. 1 shows an appearance of an entire arrangement of the ultrasonic surgical apparatus of a first embodiment. This ultrasonic surgical apparatus comprises a main unit 1, an ultrasonic treatment device (hereinafter referred to a handpiece) 2, and a foot switch 3. The handpiece 2 and the foot switch 3 are physically and electrically connected to the main unit 1.

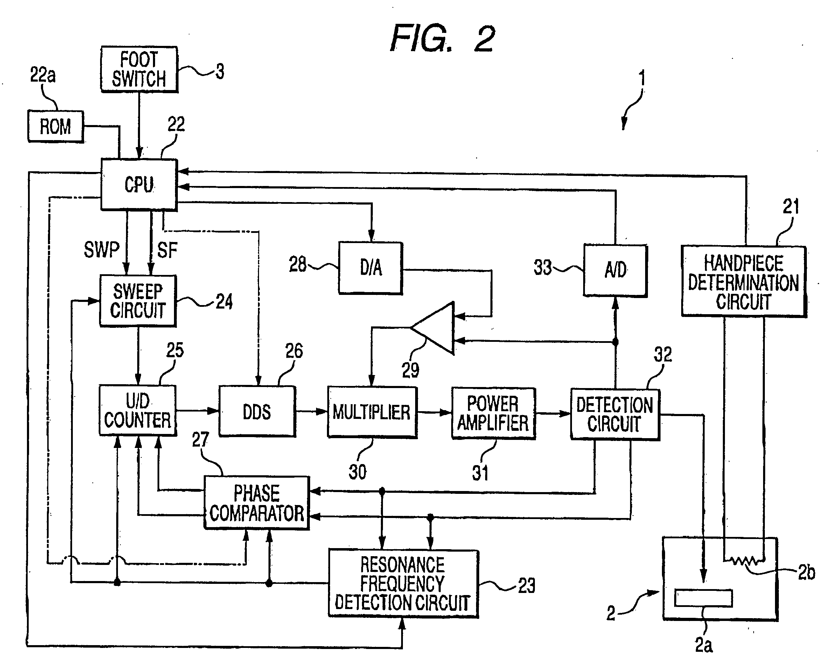

The main unit 1 drives the handpiece 2. The handpiece 2 is provided with an elongated sheath 4, with a treatment device 5 being provided at a tip thereof and an operating portion 6 being provided at a base (portion placed in an operator's hand) thereof. A case 7 for accommodating an ultrasonic transducer 2a (see Fig. 2), and an operation handle 8 are provided at the operating portion 6. The ultrasonic transducer 2a is adapted to generate mechanical vibration (longitudinal vibration) in response to a supplied current (drive current as will be described later). This mechanical vibration is also referred to ultrasonic vibration. This vibration energy is converted into frictional heat at a portion to be treated of a subject.

Inside the sheath 4, an ultrasonic probe 9 is disposed to transfer the ultrasonic vibration generated by the ultrasonic transducer 2a to the treatment device 5. A tip of this probe 9 is exposed from a tip of the sheath 4. Further, a gripper 10 which is opened/closed by a driving power with respect to the tip of the probe 9 is provided at the treatment device 5. The gripper 10 is coupled to a tip of the sheath 4 so as to enable pivotal movement thereof about a pivot pin. As is well known, an arrangement is so made that, by operating the operation handle 8, the gripper 10 is driven to open/close with respect to the tip of the probe 9, so that a living tissue portion can be gripped between the probe 9 and the gripper 10.

As shown in Fig. 1, the main unit 1 is provided, at its front face, with a front panel 11 which is provided with a power switch 12, an operation display panel 13, and a connecting portion (hereinafter referred to as a handpiece connecting portion) 14 for connecting the handpiece 2 thereto.
Among them, the handpiece connecting portion 14 is detachably connected with a connector cable 15 which is connected to the handpiece 2. Specifically, one end of the connector cable 15 is connected to the operating portion 6 of the handpiece 2, and a connector 16 disposed at the other end of the connector cable 15 is detachably connected to the handpiece connecting portion 14.

The operation display panel 13 of the main unit 1 is provided with:
a setting switch 17 (which functions as ultrasonic output setting means) for setting or changing the magnitude of ultrasonic output (i.e., vibration energy of the ultrasonic transducer 2a), that is, an amplitude value, in effecting ultrasonic treatment; and a display 18 for digitally displaying the magnitude of ultrasonic output set at the setting switch 17. Among them, the setting switch 17 includes an output increase switch 17a and
an output decrease switch 17b for increasing and decreasing, i.e. for changing, the magnitude of ultrasonic output.
It should be understood that, in the present embodiment, the magnitude of ultrasonic output is described as being its ratio to 100% output, and setting or changing of the magnitude of ultrasonic output is described as setting or changing its ratio to 100% output.

As described above, the foot switch 3 is connected to the main unit 1. The foot switch 3 has pedal member 3a. Thus, in response to the stepping operation of an operator onto the pedal member 3a, a control signal is outputted to the main unit 1 from the foot switch 3 to effect on/off control of an output of the ultrasonic vibration from the ultrasonic transducer.

Fig. 2 is a block diagram showing an electric circuit configuration of the ultrasonic surgical apparatus. As shown in Fig. 2, the main unit 1 has a circuit unit consisting of various electric circuits. Respective electric circuit portions of the handpiece 2 and the foot switch 3 are electrically connected to this circuit unit.
The aforementioned ultrasonic transducer 2a and a resistor 2b for determining a type (shape, size, material, etc.) of the handpiece 2 functioning as a treatment device, are provided inside the handpiece 2.

Among them, the ends of the resistor 2b are electrically connected to the main unit 1 through wires and the connector cable 15. The main unit 1, as shown in Fig. 2, is provided with a handpiece (HP) determination circuit 21 to which the resistor 2b is electrically connected. The HP determination circuit 21 detects resistance of the resistor 2b, and outputs a handpiece-type signal indicative of the type of a handpiece based on a detected resistance. The main unit 1 is also provided with a central processing unit (hereinafter referred to as a CPU) 22. Such a handpiece-type signal is also transferred to the CPU 22. According to the type of the handpiece 2, a maximum voltage, driving frequency and the like of a drive current (current supplied to the transducer 2a (or may be referred to as a current which the main unit 1 outputs to the transducer 2a)) are differentiated. The drive current serves as a drive signal to be fed to the transducer 2a. Thus, the CPU 22 is adapted to control various kinds of circuits, so that a suitable drive current is supplied to the handpiece 2 based on the received handpiece-type signal.
The HP determination circuit 21 and the CPU 22 constitute a principal part of determination means for determining the type of a handpiece. The function of determining a handpiece type is realized by this determination means.

In addition to the function of determining a handpiece type as described above, the main unit 1 has a resonance frequency detecting function, a PLL (phase-locked loop) function and a constant current supplying function.
In order to achieve these functions, the main unit 1 is provided with the following electric circuit components, in addition to the aforementioned HP determination circuit 21 and the CPU 22. Particularly, the main unit 1 further comprises a ROM 22a connected to the CPU 22, resonance frequency detection circuit 23, sweep circuit 24, up/down counter (hereinafter referred to as a U/D counter) 25, direct digital synthesizer (hereinafter abbreviated to DDS) 26, phase comparator 27, digital/analogue converter (hereinafter referred to as a D/A converter) 28, comparator 29, multiplier 30 serving as a modulation member, power amplifier 31, detection circuit 32 and analogue/digital converter (hereinafter referred to as an A/D converter) 33.
Among them, the ROM 22a is a memory for storing waveform pattern data of a drive current supplied to the transducer 2a. It should be understood that a RAM, not shown, is connected to the CPU 22, and that programs for performing various controls are stored in the ROM 22, so that the CPU 22 can perform the programs that have been read out from the ROM 22a using the RAM.

Such circuits as the resonance frequency detection circuit 23, the sweep circuit 24, the phase comparator 27, the D/A converter 28 and the A/D converter 33 are electrically connected to the CPU 22.

Further, the resonance frequency detection circuit 23 is electrically connected not only to the CPU 22, but also to the sweep circuit 24, the U/D counter 25 and the phase comparator 27. A phase signal from the detection circuit 32 mentioned above is supplied to the phase comparator 27 and the resonance frequency detection circuit 23.

A function of resonance frequency detection is described first. The function of resonance frequency detection is performed immediately after commencement of, i.e. at the time of starting, output to the handpiece 2. Particularly, when the pedal member 3a is stepped on, the function of resonance frequency detection is performed.
The CPU 22 permits the resonance frequency detection circuit 23 to operate at this starting time to detect resonance frequency. In particular, at the time of starting, the CPU 22 outputs to the sweep circuit 24 a sweep starting signal SWP and a starting frequency signal SF for indicating a starting frequency FO for starting sweeping. The sweep circuit 24 sets at the U/D counter 25 a count value corresponding to the frequency FO, and varies a counter output value of the U/D counter 25 by supplying an up signal or a down signal to the U/D counter 25 for gradually increasing or decreasing the frequency from the set count value. An output of the count value at the U/D counter 25 is supplied to the DDS 26, and a drive current from the DDS 26 is supplied to the transducer 2a as a drive signal. It should be understood that, when performing the function of resonance frequency detection, the CPU 22 outputs a control signal to the phase comparator 27 to stop signal supply to the U /D counter 25.

While the frequency of a drive current supplied to the transducer 2a is varied, the resonance frequency detection circuit 23 detects resonance frequency. Upon detection of resonance frequency, the resonance frequency detection circuit 23 outputs a PLL-on signal to the U/D counter 25 and the phase comparator 27 to turn on a PLL function. The PLL-on signal is also outputted to the sweep circuit 24 which then stops sweeping operation according to the on signal.

As described above, a principal part of resonance frequency detecting means is constituted by the CPU 22, the resonance frequency detection circuit 23, the sweep circuit 24 and the detection circuit 32 to thereby realize a resonance frequency detection function.

When resonance frequency is determined, PLL function is performed.
After the power switch 12 of the main unit 1 has been turned on, PLL function is performed to maintain the level of the resonance frequency as detected.

The detection circuit 32 detects waveforms of a drive current itself supplied to the transducer 2a and of a voltage corresponding thereto. The detection circuit 32 has a rectangular wave shaping circuit, and based on a current value and a voltage value of the drive current, outputs a rectangular wave signals ΔI and ΔV indicative of the respective waveform phases to the phase comparator 27. The phase comparator 27 then detects a phase shift between the rectangular wave signals ΔI and ΔV, and outputs an up signal or down signal according to the shifting amount, to the U/D counter 25. Accordingly, the U/D counter 25 varies the counter value supplied to the DDS 26, i.e. an oscillating circuit, so that the frequency (current frequency) of a drive current matches the detected resonance frequency.

In this way, the PLL function locks the frequency of a drive current supplied to the transducer 2a at a resonance frequency detected by the resonance frequency detection circuit 23, and controls the frequency so as to match the resonance frequency.

As described above, the CPU 22, the U/D counter 25, the DDS 26, the phase comparator 27 and the detection circuit 32 constitute a principal part of PLL means to achieve the PLL function.
Thus, the CPU 22 sets a predetermined value at the U/D counter 25 based on a resonance frequency detected by the resonance frequency detection function and locked by the PLL function. The DDS 26, i.e. an oscillating circuit, then outputs a predetermined frequency based on the set value. Specifically, the DDS 26 outputs an AC (alternating current) signal having a waveform according to a count value from the U/D counter 25, e.g., an AC signal having a sine waveform whose maximum amplitude is 5V (frequency is 27kHz, for example). The multiplier 30 then multiplies this signal with an amplitude modulating signal imparted to the multiplier 30 to modulate the amplitude. This amplitude-modulated AC signal is then amplified in power by the power amplifier 31 to turn into the aforementioned drive signal (drive current), which is then supplied to the transducer 2a through the detection circuit 32.
At this time, the detection circuit 32 detects a current waveform of the drive current itself as a drive signal to be supplied to the transducer 2a, and also detects a voltage waveform of the drive signal in terms of voltage, as well as an absolute value of the current.

In other words, the detection circuit 32 monitors the drive signal as a drive current and supplies a signal corresponding to an absolute value of the drive current to the A/ D converter 33 and the comparator 29. The A/D converter 33 then supplies the absolute value data of the detected drive current to the CPU 22. A drive signal value set at the setting switch 17 of the front panel 11 by an operator has been outputted to the D/A converter 28 by the CPU 22, and the D/A converter 28 supplies an analogue signal of the set value to the comparator 29. The comparator 29, i.e. a differential amplifier, supplies a signal to the multiplier 30 in accordance with the difference between the supplied set value and the detected absolute value of the drive current (amplitude modulating signal). Thus, amplitude modification as described above is performed in the multiplier 30.
In this regard, a signal fed from the CPU 22 through the D/A converter 28 serves as a reference value. This reference value can be controlled by various waveform patterns as will be described later. These waveform patterns are set so that amplitude of a drive signal to be supplied to the transducer 2a can be changed by an adequate mode relative to a time base. In other words, instead of a drive signal that has conventionally had a temporally invariable amplitude, production of a drive signal having a temporally variable amplitude has been enabled.
It should be understood that the DDS 26, the multiplier 30, and the power amplifier 31 constitute drive signal output means.

Constant current supplying function is described hereunder. The circuit configuration of a principal part related to the amplitude modulation described above also serves as a circuit configuration for realizing this constant current supplying function. Specifically, when a living tissue portion is gripped (held) between the probe 9 of the handpiece 2 and the gripper 10, impedance of the transducer goes up, and thus in turn a current goes down, which unavoidably disables desired treatment. In order to prevent this, an arrangement is made such that the comparator 29 supplies a signal to the multiplier 30 according to a difference between a supplied set value and a detected absolute value of a drive current, and that the multiplier 30 multiplies the signal with a signal from the DDS 26 to maintain the amplitude of the drive current at a set value.

Thus, constant current supplying means is constituted of the detection circuit 32, the A/D converter 33, the CPU 22, the D /A converter 28, the comparator 29 and the multiplier 30 to realize the constant current supplying function.

With the use of the ultrasonic surgical apparatus configured as described above, treatment, such as incision, can be effected to living tissue. Further, according to the type of the handpiece 2, frequency and amplitude of a drive current (i.e., drive signal) to be supplied to the handpiece 2, i.e. the transducer 2a, are differentiated. Accordingly, in the ultrasonic surgical apparatus, when the connector cable 15 of the handpiece 2 is connected to the main unit 1, the CPU 22 disposed in the main unit 1 reads a resistance value of the resistor 2b incorporated in the handpiece 2 and determines the type of the handpiece 2 based on the read resistance value. Furthermore, the CPU 22 can adequately effect incision treatment according to the type of the handpiece 2, and can supply an amplitude-controlled drive current to a transducer so that the heat at the time of treatment is not raised excessively high. In other words, the degree of generation of frictional heat at a portion of a subject being held, can be controlled by the ultrasonic vibration of the transducer 2a.

Hereinafter is described an example of a waveform of a drive current as a drive signal which is supplied to the transducer 2a of the handpiece 2 in an ultrasonic surgical apparatus.
If a conventional method is applied, an ultrasonic surgical apparatus is operated such that, when an operator steps on a pedal of the foot switch 3, for example, supply of a drive current to the handpiece 2 is started with constant amplitude, and when the operator stops stepping on the pedal, the supply of the drive current is stopped. The amplitude of the drive current is constant from the start to the stop of supply.
On the other hand, the main unit 1 of the ultrasonic surgical apparatus according to the present embodiment supplies a drive current given with predetermined modulation to the handpiece 2 when an operator steps on a pedal of the foot switch 3. In particular, in the present embodiment, an amplitude-modulated drive current is supplied to the handpiece 2.

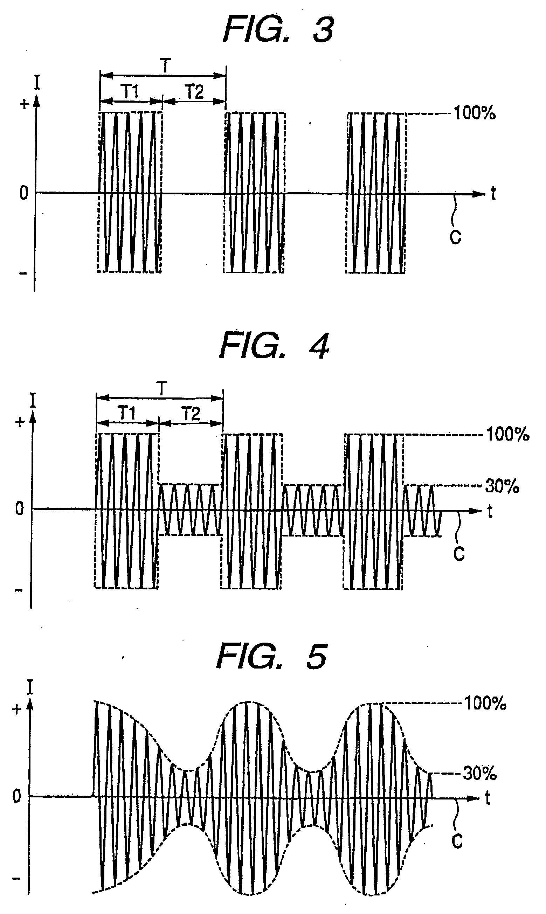

With reference to Figs. 3 to 23, various examples of waveform patterns applicable to the ultrasonic surgical apparatus according to the present embodiment are described hereunder. These waveform patterns indicate modulation variations in the drive current to be supplied to the transducer 2a of the handpiece 2. These waveform patterns may be preset or selected for each use.
It should be understood that the waveform patterns described hereunder are of AC current, and thus, in the figures, the waveform patterns are created by modulating the amplitude of an AC signal (current signal) having a frequency, for example, of 27KHz, centering on a central line C at which amplitude is 0 (zero).

Fig. 3 shows an example of a first waveform pattern of a drive current. During one period, i.e. one cycle T, of a frequency, for example, of 1 KHz, a period T1 and a period T2 are repeated, where the period T1 represents a 100% output of set amplitude, and the period T2 represents a 0% output of set amplitude. Fig. 4 shows an example of a second waveform pattern, which is rectangle, of a drive current in which a period T1 of 100% output of set amplitude and a period T2 of 30% output of set amplitude are repeated. This rectangular waveform includes the output period T2, which is low but not zero, and thus has an advantage, from the viewpoint of PLL control, that it can be readily created. Fig. 5 shows an example of a third waveform pattern, which is sine wave, of a drive current having an output between 100% and 30% of set amplitude.

In order to supply the drive currents shown in Figs. 3 to 5 to the transducer 2a of the handpiece 2, the CPU 22 outputs a voltage data to the D/A converter 28, the voltage data corresponding to an amplitude waveform pattern of a current value preset by an operator or preset according to the type of the handpiece 2. The D/A converter 28 supplies a signal corresponding to the value of the received waveform pattern data to the comparator 29. The comparator 29 then supplies an output signal to the multiplier 30, according to the difference between the set value of the waveform pattern data and an absolute value of a detected drive current. The multiplier 30 multiplies the output signal with a signal from the DDS 26, by which amplitude-modulated drive currents having waveform patterns as shown in Figs. 3 to 5 are created, in which amplitude varies relative to a time base.

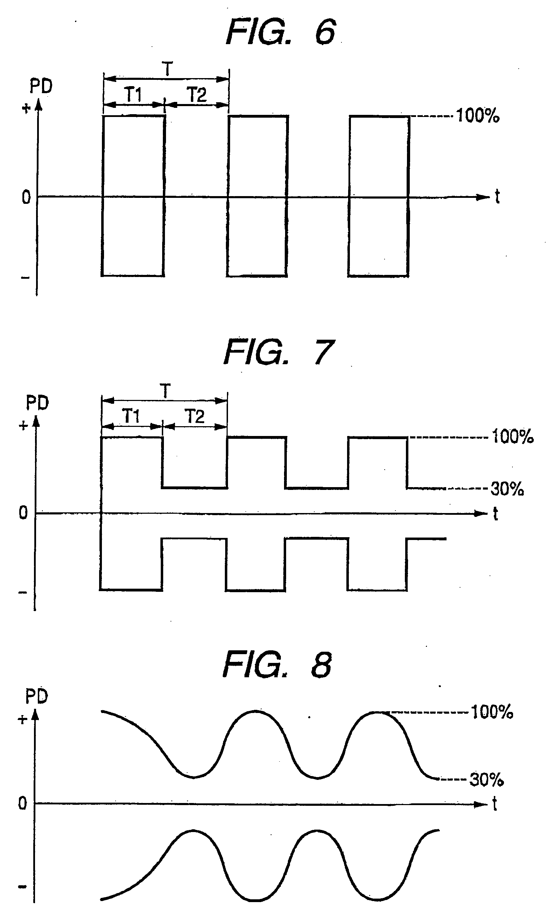

Figs. 6 to 8 show examples of waveform pattern data PD which are outputted from the CPU 22 to the D/A converter 28 to output the respective drive currents shown in Figs. 3 to 5. Each of the waveform patterns has continuous multiple pulses of a predetermined duty ratio. In each of the figures, the horizontal axis represents a time base and the vertical axis represents a set value of a drive current, i.e. a set value of a maximum output. The set value of a drive current indicates a ratio to 100% output of current supplied to the handpiece 2 from the main unit 1.
Thus, since a waveform pattern is set so that a set value is varied with time, a current signal having specific frequency, for example, of 27KHz is modulated in amplitude so as to be suppressed to the level of set value according to the waveform pattern, and supplied to the transducer 2a of the handpiece 2. The CPU 22 and the D/A converter 28 constitute a principal part of modulating means for effecting modulation of a drive current.

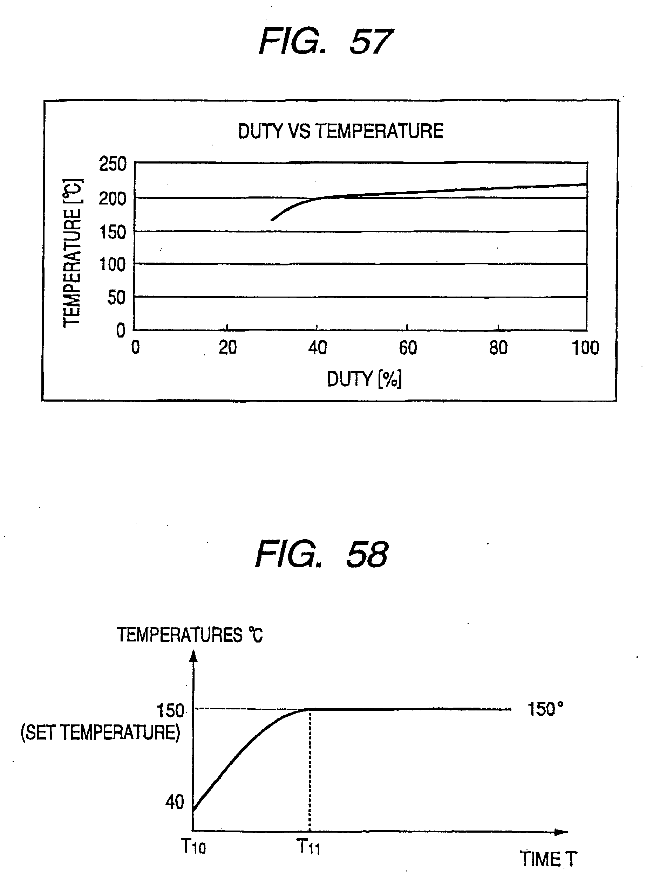

It should be understood that the duty ratio "T1/(T1+T2)" may be 5% to 100%, preferably, 5% to 50%, and that the period T may be 0.1 to 1 second, preferably, 0.4 to 1 second. These numerical values are based on the experiments carried out by the inventors of the present invention.
One example of the results of the experiments is shown in Fig. 57. This figure shows a relation between a duty ratio and temperature. According to this, since temperature increases until the duty ratio is rendered to be 100%, an upper limit of the 100% duty ratio can be used. As can be seen, since saturation starts at around a duty ratio of 50%, even if the duty ratio is increased more than that, i.e. even if the energy given to a treatment device is increased by friction, temperature does not drastically increase. Therefore, an upper limit of a particularly preferable duty ratio is about 50%. Although no lower limit duty ratio is shown in Fig. 57, this is base on a confirmation that incision was not enabled for a treatment device until a duty ratio was rendered to be about 5%.

Because each pulse output of such waveform patterns has a high output period T1 having 100% output of drive current amplitude, the incision capability of the handpiece 2 is not varied. Further, because each pulse output has a low output period T2 having non-100% output of drive current amplitude, the overheating of the treatment device 5 of the handpiece 2 can be suppressed. In particular, generation of frictional heat due to ultrasonic vibration of the probe 9 can be prevented. Accordingly, even when the treatment device 5 is brought into touch with living tissue during operation, transformation is unlikely to occur in the living tissue since the temperature of the treatment device 5 is not high.

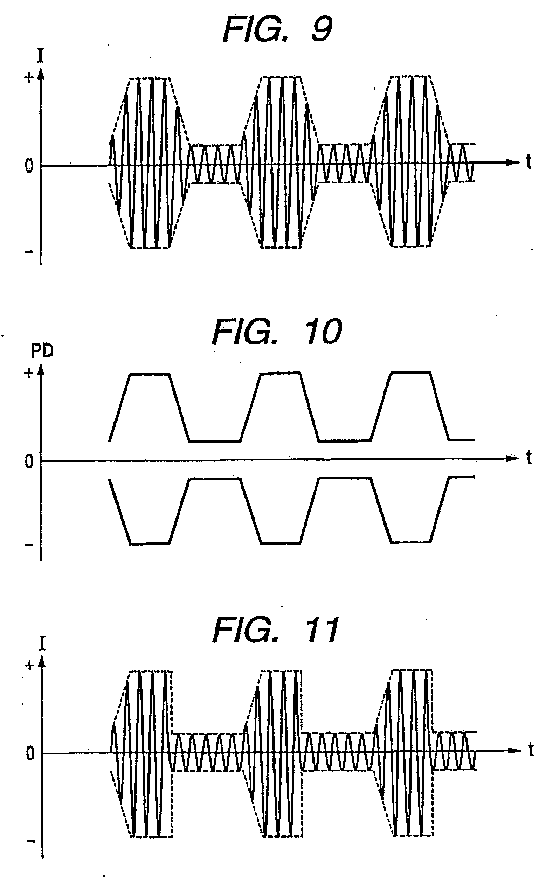

Figs. 9 to 20 show other examples of output waveforms of a drive current supplied to the handpiece 2, and waveform patterns supplied to the D/A converter 28 from the CPU 22.

Fig. 9 shows a fourth waveform pattern, i.e. a current waveform diagram, in which drive current amplitude supplied to the handpiece 2 varies along the shape of a trapezoid. For this waveform pattern, the graded portions in the trapezoids allow the PLL control to be well maintained. Fig. 10 shows a waveform pattern outputted from the CPU 22 to the D/A converter 28 for the drive current shown in Fig. 9 to be outputted. For example, a wave form pattern is outputted so that a current signal having frequency of 27KHz forms a 1KHz-period trapezoidal waveform pattern of current amplitude. Therefore, the start-up of a drive current is not abrupt but gradually goes up to a 100% level.

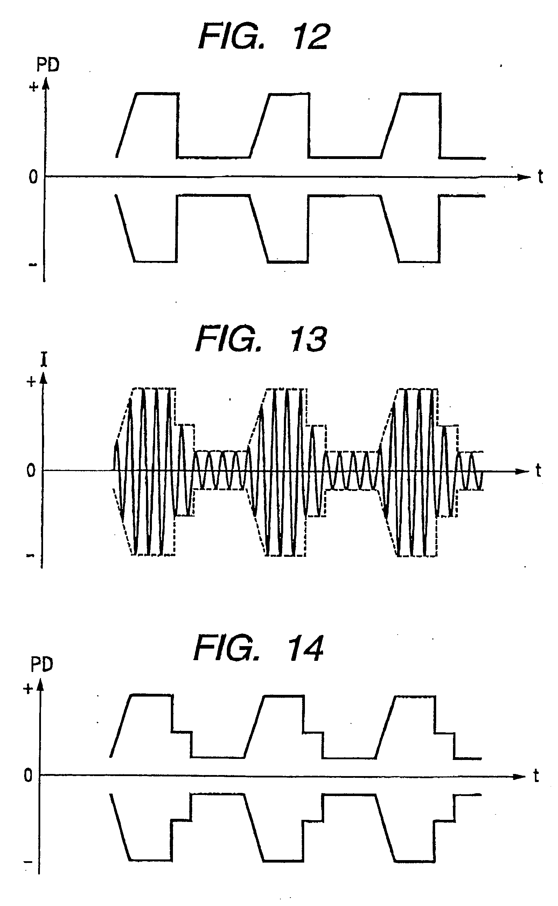

Fig. 11 shows an example of a fifth waveform pattern, i.e. a current waveform diagram, in which amplitude of a drive current supplied to the handpiece 2 varies along a trapezoidal shape different from the one shown in Figs. 9 and 10. Fig. 12 shows a waveform pattern outputted from the CPU 22 to the D/A converter 28 for the drive current shown in Fig. 11 to be outputted.

Fig. 13 shows an example of a sixth waveform pattern, i.e. a current waveform diagram, in which the amplitude of a drive current supplied to the handpiece 2 varies along a trapezoidal shape different from the ones shown in Figs. 9 to 12. Fig. 14 shows a waveform pattern outputted from the CPU 22 to the D/A converter 28 for the drive current shown in Fig. 13 to be outputted. The shapes of the waveforms shown in Figs. 13 and 14 each are the combination of a trapezoid and a rectangle.

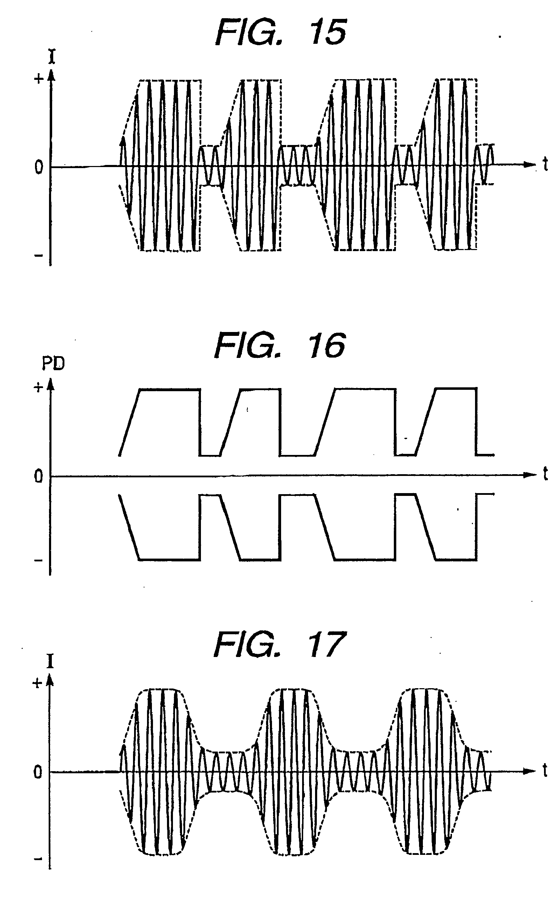

Fig. 15 shows an example of a seventh waveform pattern, a current waveform diagram, in which the amplitude of a drive current supplied to the handpiece 2 varies along a trapezoidal shape different from the ones shown in Figs. 9 to 14. Fig. 16 shows a waveform pattern outputted from the CPU 22 to the D/A converter 28 for the drive current shown in Fig. 15 to be outputted. The waveforms shown in Figs. 15 and 16 are obtained by combining a plurality of different trapezoidal waveforms into one waveform pattern, with one combination of the waveforms as one cycle being repeatedly outputted.

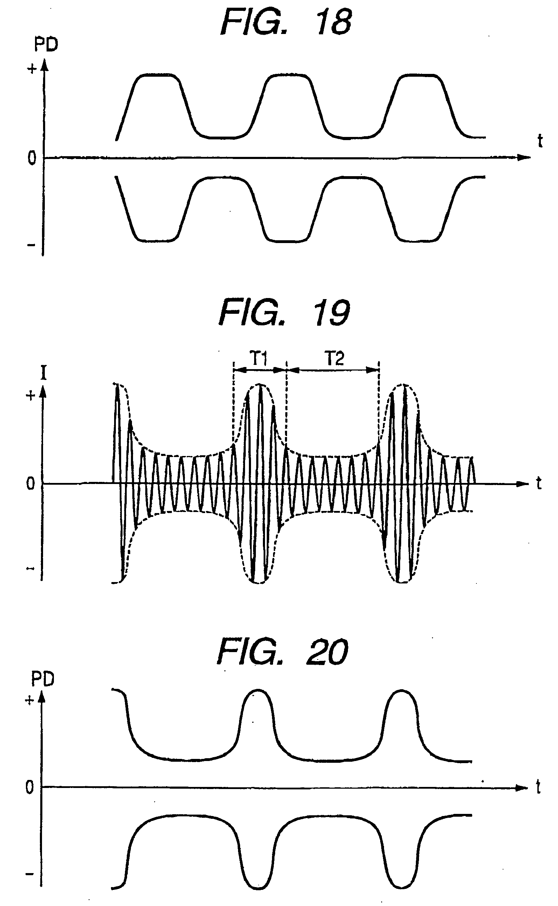

Fig. 17 shows an example of an eighth waveform pattern, i.e. a current waveform diagram, in which the amplitude of a drive current supplied to the handpiece 2 varies along a rounded trapezoidal shape based on the trapezoidal shape shown in Fig. 9. Fig. 18 shows a waveform patter outputted from the CPU 22 to the D/A converter 28 for the drive current shown in Fig. 17 to be outputted.

Fig. 19 shows an example of a ninth waveform pattern, a current waveform diagram, in which the amplitude of a drive current supplied to the handpiece 2 varies along a modified sine waveform. For this waveform pattern, PLL control can be readily performed, and a low output period T2 can also be readily ensured. Fig. 20 shows a waveform pattern outputted from the CPU 22 to the D/A converter 28 for the drive current shown in Fig. 19 to be outputted.

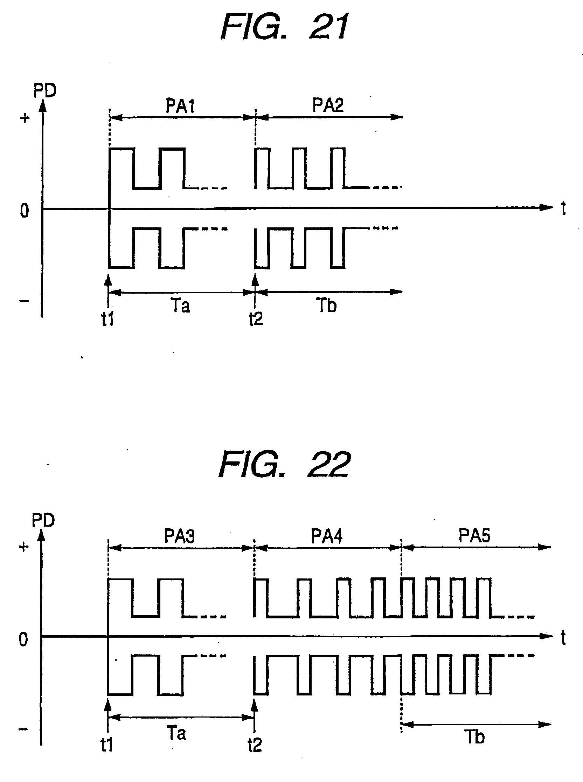

As shown in Figs. 21 to 23, after starting treatment, a waveform pattern may be changed between the one in an initial predetermined period Ta and the one in a subsequent period Tb. Figs. 21 to 23 show examples of the waveform patterns in which waveform patterns are changed in mid-course.
A waveform pattern is changed in mid-course depending on conditions, such as the contents of treatment carried out by an operator, and the way of using treatment devices. For example, Fig. 21 shows a tenth waveform pattern, in which, during a period Ta, an initial waveform pattern PA1 immediately after the foot switch 3 has been depressed presents, in one cycle "T", a long waveform pattern having a high-output period T1 of Fig. 7, and after expiry of the period Ta, another period Tb follows thereto. During the period Tb, a combination waveform pattern PA2 is presented in which the high-output period T1 of Fig. 7 is rendered to be shorter. In short, in the continuous waveform of multiple pulses, a duty ratio is changed in mid-course.

Fig. 22 shows an eleventh waveform pattern in which, during a period Ta, an initial waveform pattern immediately after the foot switch 3 has been depressed presents, in one cycle "T", a long waveform pattern PA3 having a high-output period T1 of Fig. 7. After expiry of the period Ta, a period PA4 follows thereto in which the high-output period T1 is short and constant and one cycle T is gradually shortened. After expiry of this period, a waveform pattern PA5 follows in which the high-output period T1 of Fig. 7 is short. Specifically, in a period between the period Ta and the period Tb, there is presented the waveform pattern PA4 in which one cycle T is gradually shortened. In particular, in a continuous waveform of multiple pulses, the length of one cycle, as well as a duty ratio, is changed in mid-course.

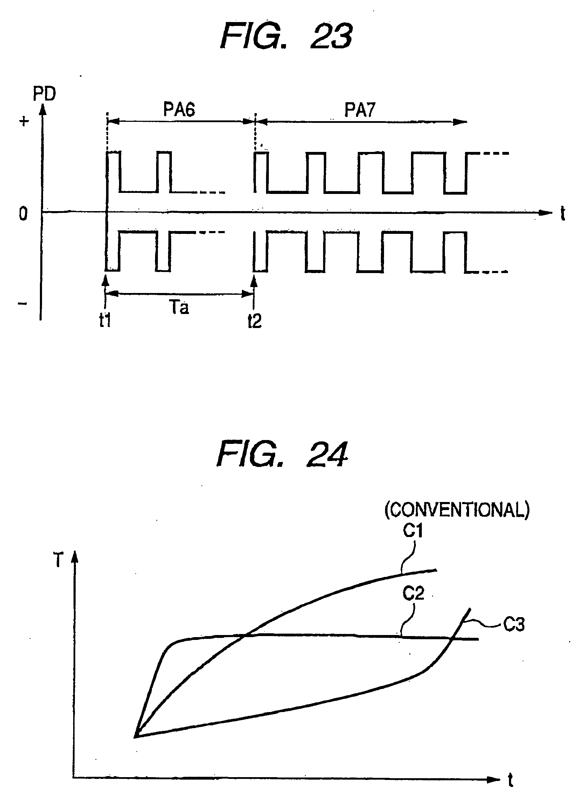

In a twelfth waveform pattern shown in Fig. 23, in one cycle T, a waveform pattern PA6 is presented with a high-output period T1 being constant during the aforementioned initial period Ta. After expiry of this period Ta, the high-output period T1 gradually increases with one cycle T remaining constant. That is to say, a waveform pattern PA7 is presented
in which the period T2 is gradually reduced. Specifically, in the continuous waveform of multiple pulses, the duty ratio is changed in mid-course.

The pattern shown in Fig. 23, for example, is preferable in case coagulation treatment is effected at low temperature with the waveform pattern PA6 of the initial period Ta, and incision treatment is thereafter effected by drastically raising temperature with the waveform pattern PA7.

Fig. 24 shows an example of temperature variation at the treatment device 5 of the handpiece 2. In Fig. 24, temperature variation of a conventional handpiece results in as shown by a curve C 1 in which temperature gradually increases with time.

Contrarily, for the cases shown in Figs. 21 and 22, temperature increase of a handpiece can be suppressed as shown by a curve C2 in Fig. 24. For the case shown in Fig. 23, temperature is initially low but can be drastically increased in mid-course as shown by a curve C3. The temperature variation of the curve C3 is preferable, for example, in case living tissue, such as a blood vessel, is initially coagulated at low temperature, and then incised by drastically raising temperature.

Setting of the various pattern data PD described above have been automatically carried out according to the type of the handpiece 2 determined by the HP determining circuit 21, however, an operator may often wish to finely control a waveform pattern or to change the setting to another setting value. In such cases, an operator may allow an automatically selected set value to be indicated on the display 18 with a function switch, not shown, of the front panel 11 shown in Fig. 1, and then may change the indicated set value by operating the output increase switch 17a or the output decrease switch 17b. The amplitude of a drive current is then controlled so that output is performed along the waveform pattern determined based on the changed set value.

In the description provided above, the waveform pattern data has been stored in the ROM 22a connected to the CPU 22, however, the data may be stored in a rewritable memory, such as a flash memory.

It should be understood that, according to the type of the handpiece 2, modification may be made in the process for changing the maximum amplitude, i.e. 100% output, of a drive current and the frequency of the drive current. For example, an arrangement may be made wherein a waveform pattern data PD of a drive current to be supplied to the handpiece 2 is recorded in advance into a ROM incorporated in the handpiece 2, and the ROM data is transferred to the main unit 1 to allow the main unit 1 to control the drive current based on the ROM data.

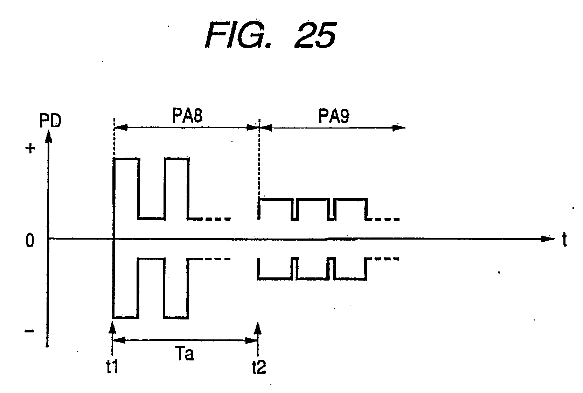

Moreover, in the waveform pattern examples described above, either amplitude or a duty ratio has been changed, however, as shown in Fig. 25, a waveform pattern may be such that a drive current and amplitude are simultaneously changed (thirteenth waveform pattern).

Specifically, as shown in Fig. 25, a waveform pattern PA8 is presented in which the high-output period T1 is constant at a predetermined first duty ratio in one cycle T, during the initial period Ta described above. After expiry of this period Ta, a waveform pattern PA9 follows in which the length of one cycle T is the same as or different from the initial period Ta. In the pattern PA9, a second duty ratio different from the first duty ratio is imparted, and the level of amplitude during the high-output period T1 is different from the one in the pattern PA8. Accordingly, a drive current after time t2 is different from the drive current before time t2 in its amplitude and duty ratio.
As described above, according to the ultrasonic surgical apparatus of the first embodiment, ultrasonic treatment (incision, coagulation, etc.) can be effected based on a drive current which is controlled in its amplitude so that frictional heat due to the vibration of the probe 9 may not be excessively increased during the treatment. Thus, unlike the conventional ultrasonic treatment based on a drive current having constant amplitude, the inventive device is capable of adequately controlling heat required for the treatment. For this reason, such inconvenience can be avoided as the occurrence of undesired incision prior to coagulation due to the transfer of heat to the inside of a portion to be treated. Particularly, while ensuring amplitude of a drive current at a required level, a time zone, in which amplitude is to be reduced, is set by an adequate mode. The time zone where the amount of heat to be generated is suppressed, efficiently functions by permitting previously generated heat to be sufficiently diffused to the inside. Thus, heat transfer to a treatment device is suppressed to control the timing of incision and coagulation, so that incision can be performed while coagulating the portion to be treated. In particular, incision can be performed substantially in parallel with coagulation.
In addition, incision capability based on the required level of amplitude can also be sufficiently ensured. That is, a good balance can be achieved between suppression of heat generation in a treatment device and retaining incision capability.
Furthermore, according to the present embodiment, incision treatment can be adequately effected according to the type of the handpiece 2.

### (Second Embodiment)

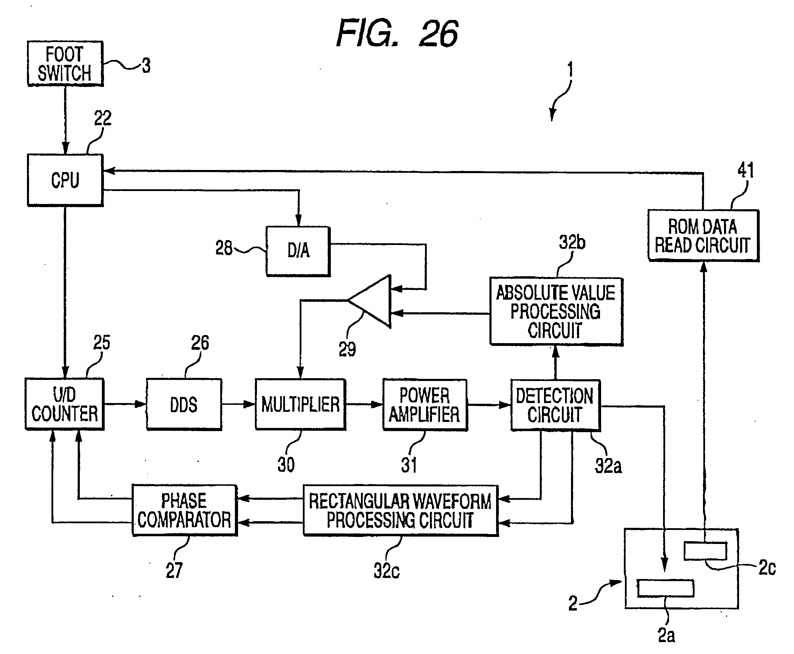

With reference to Fig. 26, a second embodiment of the ultrasonic surgical apparatus according to the present invention is described.
Fig. 26 is a block diagram illustrating an electric circuit configuration of an ultrasonic surgical apparatus, which is a modification of the configuration shown in Fig. 2. In this circuit configuration a ROM recorded with waveform pattern data is incorporated into a handpiece 2. The same components as in Fig. 2 are referred to by the same reference numerals, and description therefor is omitted.
In the circuit configuration shown in Fig. 26, a detection circuit 32a detects a current signal and a voltage signal, and supplies the current signal to an absolute value processing circuit 32b. The absolute value processing circuit 32b supplies an absolute value signal of the current signal to the comparator 29. Also, the detection circuit 32a supplies the current signal and the voltage signal to a rectangular waveform processing circuit 32c. The rectangular waveform processing circuit 32c then supplies rectangular waveform signals of the respective current signal and voltage signal to the phase comparator 27.

The handpiece 2 is incorporated with a ROM 2c, and the main unit 1 is provided with a ROM data read circuit 41 which is connected to the ROM 2c through the connector cable 15. The ROM data read circuit 41 is connected to the CPU 22 to supply the waveform pattern data PD stored in the ROM 2c thereto. The waveform pattern data PD stored in the ROM 2c are the ones shown in Figs. 6-8, 10, 12, 14, 16, 18, 20, 21-23 and 25. Accordingly, the amplitude of a drive current is modulated when the CPU 22 supplies the D/A converter 28 with a set value data according to a waveform pattern data PD.

In addition to the treatment devices utilizing ultrasonic waves, other treatment devices may sometimes be used together. In consideration of such cases, a ROM incorporated into a treatment device may be made capable of recording thereinto an information data of "No modulation".

It should be understood that, alternatively, an arrangement may be so made that, depending on the contents or the like of surgery, an operator can finely control a waveform pattern determined in advance according to the type of the handpiece 2. The waveform pattern data PD have been set and recorded in the ROM 22a or 2c according to respective types of the handpiece 2. Thus, when the handpiece 2 is connected to the main unit 1, the CPU 22 indicates on the display 18 a minimum value of a drive current and a duty ratio determined according to the type of the handpiece 2.
Accordingly, an operator can finely control and change the displayed individual values by operating the switches 17a and 17b. Then, by inputting a command (not shown) for registering a set value, a waveform pattern can be stored in the RAM of the CPU 22. The display 18 serves as one for indicating and setting a minimum value of a drive current and a duty ratio.

For example, for the waveform patterns shown in Figs. 6 to 8, an operator may temporarily permit a preset minimum value ratio in the period T2 to be indicated on the display 18 by inputting a predetermined command to the CPU 22. Then, the operator may change and finely control the minimum value ratio by operating the switches 17a, and 17b. Further, an operator may temporarily permit a preset duty ratio to be indicated on the display 18 by inputting a predetermined command to the CPU 22. Then, the operator may change and finely control the duty ratio by operating the switches 17a and 17b. The change of a duty ratio involves, for example, a ratio (%) of the period T1 to one cycle, or a ratio (%) of the period T2 to one cycle. For a sine waveform pattern as well, an arrangement may be so made that a duty ratio can be changed. For example, as shown in Fig. 19, a duty ratio can be changed by modifying a sine waveform so that the ratio of the high-output period (T1) would not be 50%. The high-output period T1 is a period in which a maximum value of a current is not less than a predetermined value.

In the examples described above, the preset waveform pattern data PD, or finely controlled waveform pattern data PD according to respective types of the handpiece 2 have been supplied to the CPU 22. Alternatively, an arrangement may be so made that an operator can optionally set a waveform pattern data PD depending on the contents or the like of surgery.

### (Third Embodiment)

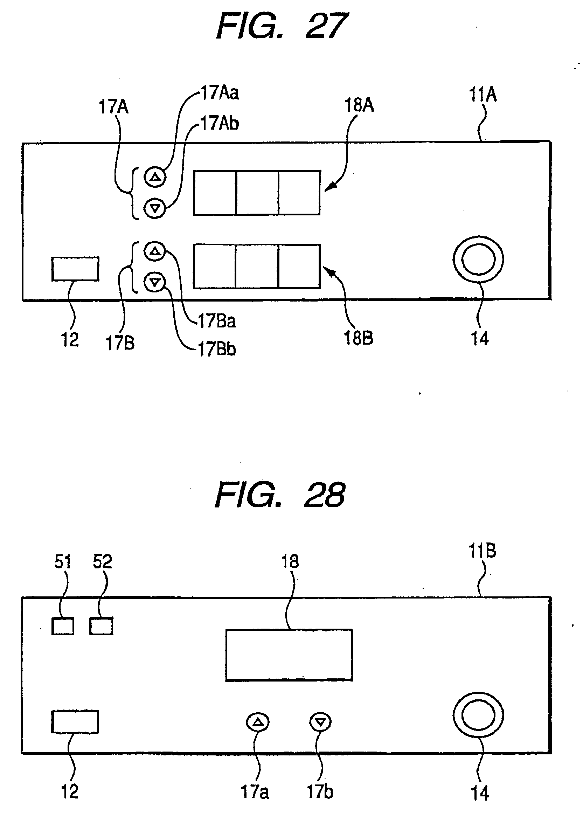

With reference to Fig. 27, a third embodiment of the ultrasonic surgical apparatus according to the present invention is described.
Fig. 27 shows another example of a front panel for an operator to set a waveform pattern data PD.
A front panel 11A shown in Fig. 27 is provided with a pair of digital displays 18A, 18B, and a pair of switches 17A, 17B for increasing and decreasing output, which correspond to the respective digital displays. The switches 17A, 17B, respectively, comprise switches 17Aa, 17Ab and switches 17Ba, 17Bb for increasing and decreasing output. The display 18A is a display for indicating and setting a minimum output value, i.e. a ratio (%) of the minimum output value to a 100% maximum output value. An operator can set a desired minimum value by depressing the switches 17Aa, 17Ab observing a value indicated on the display 18A. In a similar fashion, the display 18B is a display for an operator to set a one-cycle duty ratio. An operator can set a desired duty ratio by depressing the switches 17Ba, 17Bb observing a value indicated on the display 18B.

For example, since a maximum value is determined according to the type of the handpiece 2, an operator may allow the display 18A to indicate a ratio of "50" (%) as a minimum ratio of current amplitude to a maximum output value. Then, the operator may allow the display 18B to indicate a ratio of "60" (%) as a duty ratio, i.e. a ratio of maximum output to one cycle. When a command (not shown) for registering set value is inputted in this state, the waveform pattern data PD can be stored in the RAM of the CPU 22.
In this way, an operator may be able to optionally set a waveform pattern data PD of a drive current of the handpiece 2, depending on the contents or the like of surgery.

### (Fourth Embodiment)

With reference to Figs. 28 to 31, a fourth embodiment of the ultrasonic surgical apparatus according to the present invention is described.
An arrangement may be made such that an operator can optionally select a waveform pattern data PD of a drive current of the handpiece 2 depending on the contents or the like of surgery. Figs. 28 to 30 illustrate an example in which a waveform pattern is optionally selected.

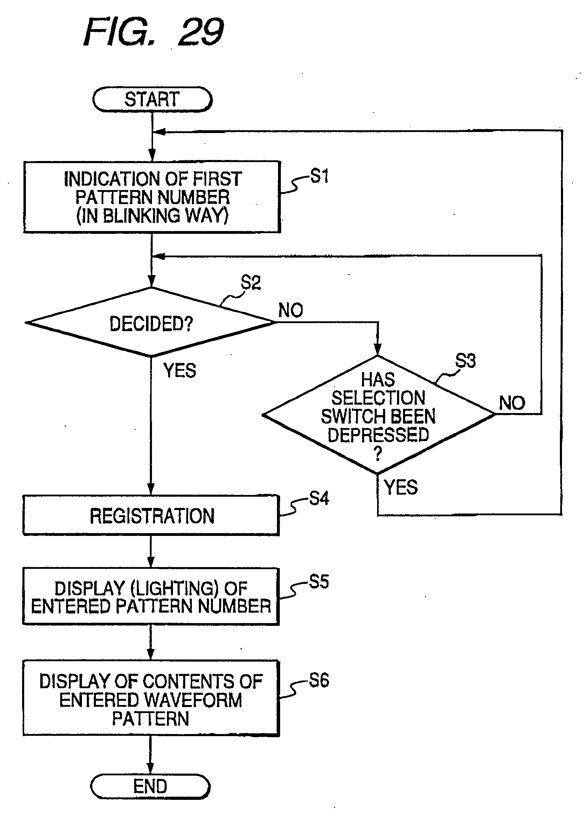

Fig. 28 shows an example a front panel in case a waveform pattern is selected. Fig. 29 is a flow diagram showing an example of a process flow performed in a CPU of a main unit 1 in selecting a waveform pattern.

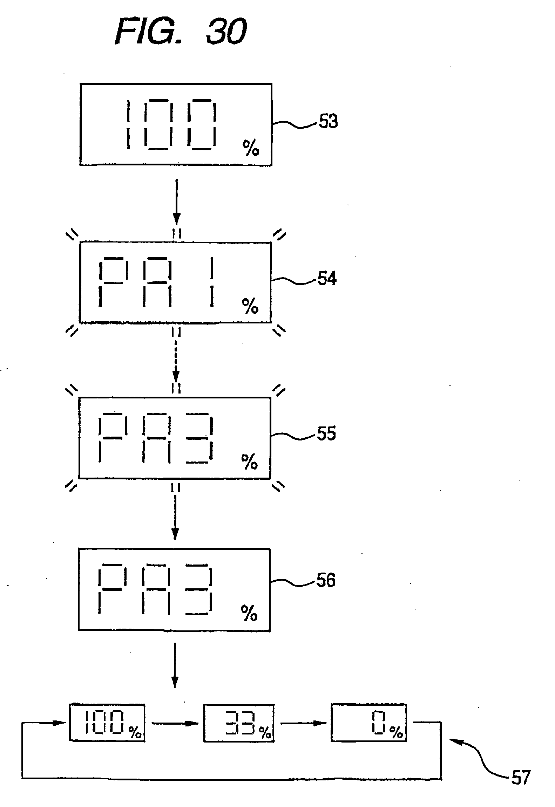

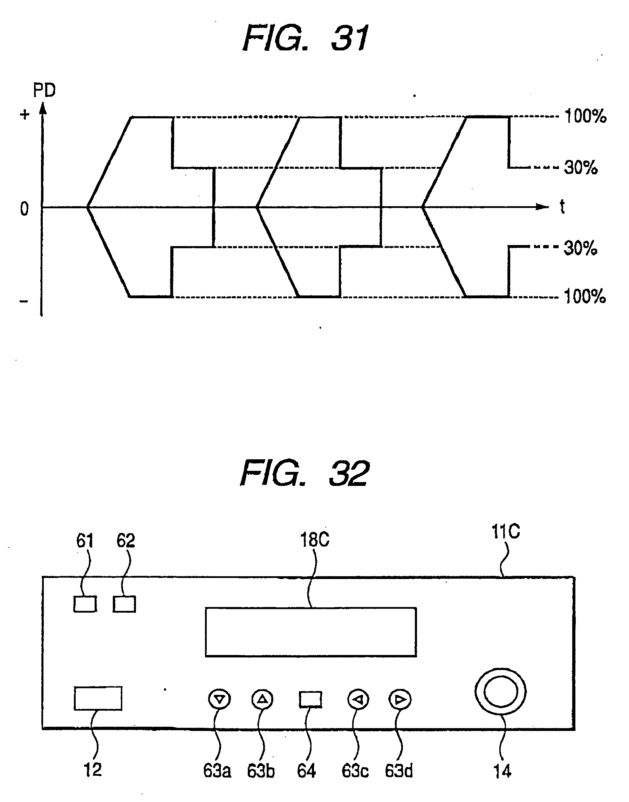

Fig. 30 shows examples of indication on the front panel in selecting a waveform pattern. Fig. 31 shows an example of a waveform pattern data (fourteenth waveform pattern data).

Similar to the front panel 11 shown in Fig. 1, a front panel 11B comprises a power switch 12, a display 18, switches 17a, 17b and a handpiece connecting portion 14. The front panel 11B further comprises a memory switch 51 serving as a switch for reading out data, and a selection switch 52 for selecting a waveform pattern.

A process flow which is performed when an operator selects a waveform pattern is described hereunder with reference to Fig. 29. When the selection switch 51 is depressed by an operator, the CPU 22 executes the process shown in Fig. 29.
When the selection switch 51 is depressed initially, a first pattern number is indicated (blinked) (step S1) from among a plurality of waveform patterns recorded on a memory, such as a ROM, according to a predetermined sequence. In this case, as shown in Fig. 30, up until the depression of the selection switch 51, a numeral "100" indicative of 100% output of a drive current is continuously lit on the display 18 (see state 53 in Fig. 30). Then, with the depression of the selection switch 51, a pattern number "PA1" is blinkingly indicated (see state 54 in Fig. 30) as the first pattern number. Further, a determination is made (step S2) as to whether or not an operator has depressed a memory switch 52, which means operator's confirmation of entry. If the memory switch 52 is not depressed, a determination is made (step S3) as to whether or not the section switch 51 has been depressed.

When the selection switch 51 is depressed, the determination at step S3 results in YES, and control returns to step S1 to blinkingly indicate a next pattern number, i.e. "PA2" in this case. When the selection switch 51 is further depressed, step S2 results in NO and step S3 results in YES, so that control again returns to step S1 to blinkingly indicate the next pattern number, i.e. "PA3" in this case. In this way, at step S1, pattern numbers of the waveform patterns stored in the ROM or the like are sequentially indicated (see state 55 in Fig. 30).

If the depression of the memory switch 52 takes place, which means an operator's confirmation of entry of a waveform pattern, step S2 results in YES. Then, a registration process is performed (step S4) for storing the pattern number in a memory, such as a RAM. Control then proceeds to step S5 in which the entered pattern number is lit on the display 18 (see state 56 in Fig. 30). After the entered pattern number is lit for a specific period of time, the contents of the entered waveform pattern data are indicated (step S6).

For example, if a selected and entered pattern number corresponds to a waveform pattern shown in Fig. 31, repeating indication as shown by state 57 in Fig. 30 is performed on the display 18. Specifically, Fig. 31 shows one pattern in which output is gradually increased from 0% to 100% for an initial period of time, the 100% output is maintained for a specific period of time, a 33% output is then maintained for a specific period of time, and a 0% output is then performed. Thus, on the display 18, indication from "100%" to "33%" and then to "0%" is repeated as shown by the state 57 in Fig. 30.
As described above, an operator can optionally select a waveform pattern of a drive current of the handpiece 2 depending on the contents or the like of surgery, while the pattern number of a selected waveform pattern is stored in a RAM. Since the waveform pattern data PD corresponding to the stored pattern number is outputted to the D/A converter 28 from the CPU 22, the handpiece 2 turns out to be the one which provides good usability for an operator.

### (Fifth Embodiment)

With reference to Figs. 32 to 34, a fifth embodiment of the ultrasonic surgical apparatus according to the present invention is described below.
An arrangement may be made such that an operator can optionally set a waveform pattern of a drive current of a handpiece 2 depending on the contents or the like of surgery.

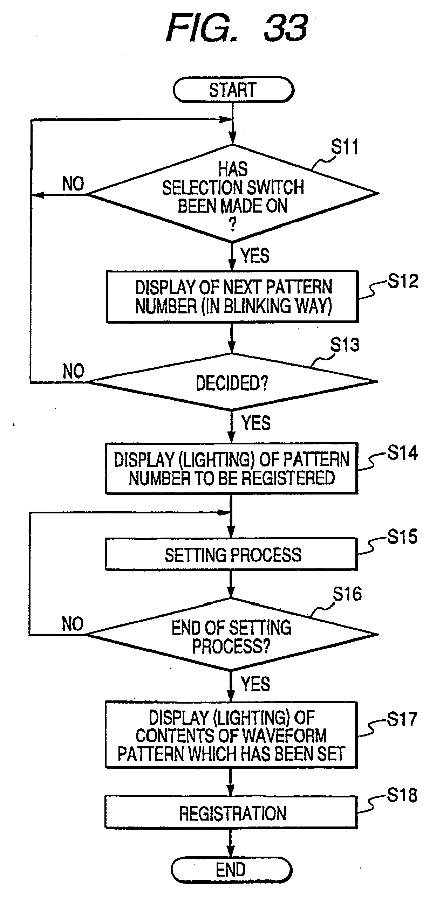

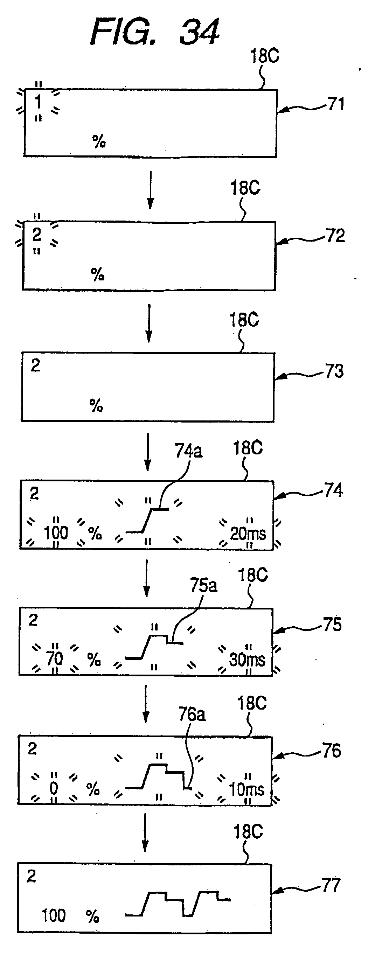

Figs. 32 to 34 illustrate an example in which a waveform pattern data is optionally set. Fig. 32 shows another example of a front panel used for setting a waveform pattern. Fig. 33 is a flow diagram showing an example of the processes performed by a CPU of a main unit 1 in setting a waveform pattern data. Fig. 34 shows examples of indication on the front panel in setting a waveform pattern data.

Similar to the front panel 11 shown in Fig. 1, a front panel 11C comprises a power switch 12 and a handpiece connecting portion 14. The front panel 11C further comprises a display 18C, a memory switch 61 serving as a switch for designating a registration number, a selection switch 62 for selecting a waveform pattern, increase/decrease switches 63a, 63b, 63c and 63d, and an entry switch 64 for registration.

A process flow for an operator to optionally set a waveform pattern is described with reference to Fig. 33. Upon depression of the memory switch 61 by an operator, a CPU 22 executes the processes shown in Fig. 33. When the memory switch 61 is initially depressed, a pattern number is indicated for which a waveform pattern data to be set is registered. At this stage, as shown by a state 71 in Fig. 34, numeral "1" is blinkingly indicated as a first pattern number.

The CPU 22 determines (step S11) first as to whether or not the selection switch 62 has been depressed, and then stands by until the selection switch 62 is depressed. With the depression of the selection switch 62, a next pattern number is blinkingly displayed at step S12 (see state 72 in Fig. 34). Then, a determination is made (step S13) as to whether or not the entry switch 64 for confirming entry has been depressed. If the entry switch 64 is not depressed, NO-determination is made at step S 13, and control then returns to step S11.

Upon depression of the entry switch 64, determination at step S 13 results in YES, so that control proceeds to step S14 to light up a registration pattern number, that is, a pattern number to be registered (see state 73 in Fig. 34).
Then, being in a state capable of performing a waveform pattern setting process, the CPU 22 executes setting process (steps S15 and S16)
in which an operator can set a waveform pattern using the switches 63a, 63b, 63c and 63d and the entry switch 64.

When the CPU 22 is in the setting process of a waveform pattern, an operator can set a waveform pattern in the following procedures. The switch 63a serves as a button for instructing output decrease, and the switch 63b serves as a button for instructing output increase. The switch 63c serves as a button for instructing decrease in output time, and the switch 63d serves as a button for instructing increase in output time.

For example, assuming that a drive current of 100% output is to be outputted initially for 20ms (millisecond which also applies to the following description), the initial output is rendered to be 100% by using the switch 63b, and the output time indicated on the display 18C is changed from 0ms to 20ms, for example, by using the switch 63d. When the entry switch 64 is depressed at this stage, the output of the first 20ms period is indicated on the display 18C as an initial waveform pattern shown by an indication 74a in Fig. 34 (see state 74 in Fig. 34).

Similarly, by using the switches 63a, 63b, 63c and 63d, the output and the output time of a second period are set. For example, when a drive current of 70% output with an output time of 30ms is set, followed by depression of the entry switch 74, a waveform pattern shown by an indication 75a in Fig. 34 is indicated on the display 18C (see state 75 in Fig. 34). Further, in the similar manner, the output and the output time of a third period are set using the switches 63a, 63b, 63c and 63d. For example, when a drive current of 0% output with an output time of 10ms is set, followed by depression of the entry switch 74, a waveform pattern as shown by a indication 76a in Fig. 34 is indicated on the display 18C (see state 76 in Fig. 34).

The setting process is carried out in this way. During the setting process (step S15), a determination is constantly made (step S16) as to whether or not the memory switch 64 has been depressed for confirming the end of pattern setting. If the memory switch 64 has not been depressed, the determination at step S16 results in NO, and control returns to step S 15.

If the entry switch 64 is depressed, the determination at step S 16 results in YES, and control proceeds to step S17 where the contents of the set waveform pattern are lit up (see state 77 in Fig. 34). Further, a registration process is performed (step S18) for storing the set waveform pattern in a RAM.

As described above, an operator can optionally set a waveform patter of a drive current of the handpiece 22 depending on the contents or the like of surgery, and the set waveform pattern is stored in a RAM. Since the stored waveform pattern data PD is outputted from the CPU 22 to the D/A converter 28, the handpiece 2 turns out to be the one providing good usability for an operator.

### (Sixth Embodiment)

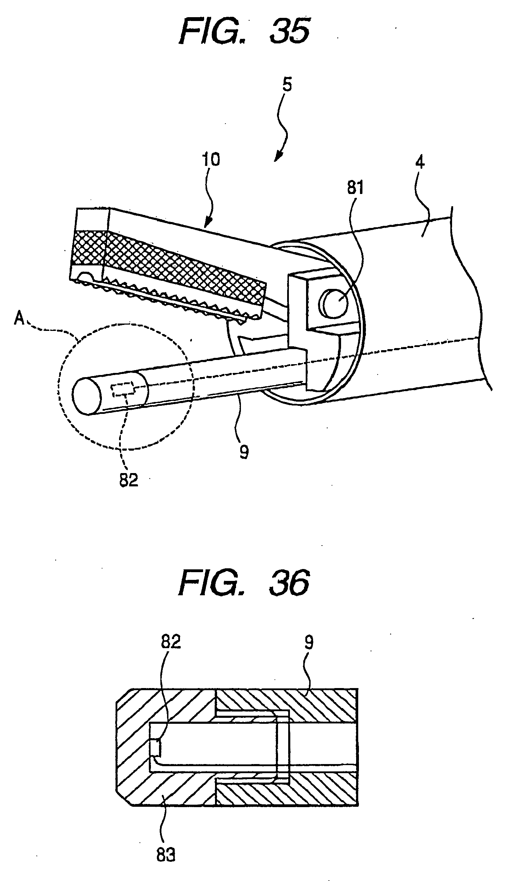

With reference to Figs. 35 to 37, a sixth embodiment of the ultrasonic surgical apparatus according to the present invention is described.
An arrangement may be so made that an amplitude-modulated current signal is outputted according to a predetermined trigger signal. Specifically, an arrangement may be so made that an operator can detect timing for using a handpiece 2 with a predetermined trigger signal, so that a predetermined amplitude-modulated drive signal (current) is outputted. Various examples of trigger signals are described hereunder.

Hereunder is described an example of a temperature sensor that can be implemented in this embodiment. In this example, an output of the temperature sensor serves as such a trigger signal. Fig. 35 is a perspective illustration of a treatment device 5 in which an output of the temperature sensor serves as a trigger signal. A probe 9 and a gripper 10 are provided at a tip of the treatment device 5. The gripper 10 is pivotally linked to a tip of a sheath 4 so as to turn about a pivot pin 81. By operating an operation handle 8, the gripper 10 is driven to open/ close with respect to the tip of the probe 9. A temperature sensor 82, such as a thermo couple, as heat detecting means is provided inside the probe 9.

Fig. 36 is a cross section of the tip of the probe 9 circled by a dotted line A in Fig. 35. As shown in Fig. 36, the temperature sensor 82 is adhered to an inner wall surface of a metal cap 83 at the tip, and is adapted to detect temperature of the probe 9.

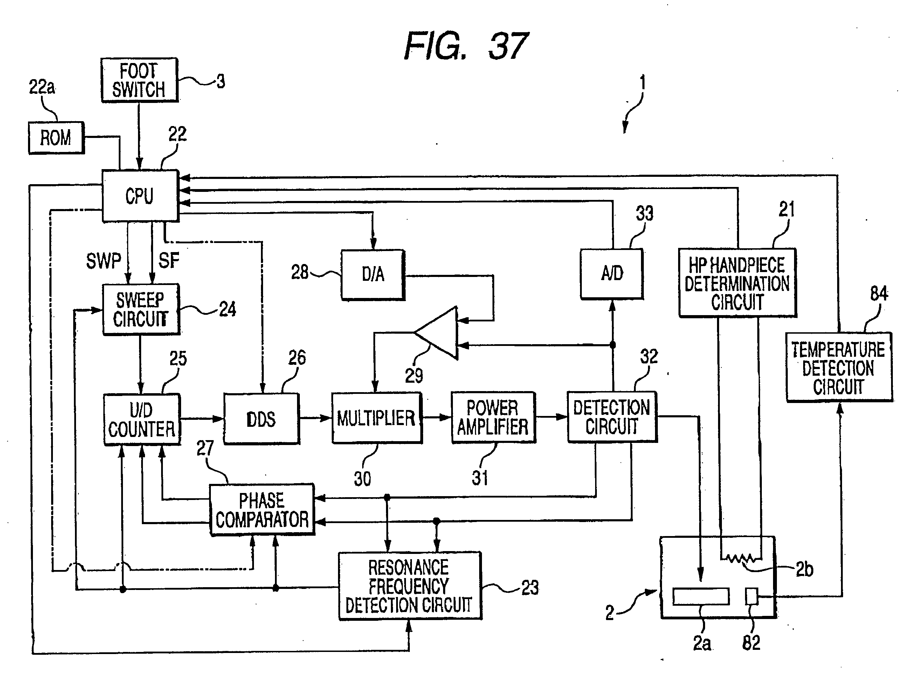

Fig. 37 is a block diagram showing a circuit configuration of a main unit 1, which is provided with a temperature detection circuit 84 for receiving a signal from the temperature sensor 82. In the figure, the components having the same configurations as those in Fig. 2 are referred to by the same reference numbers, and description therefor is omitted. The only difference from the configuration shown in Fig. 2 is that, in the present embodiment, the temperature detection circuit 84 is provided to the main unit 1, and that temperature data detected by the temperature detection circuit 84 is arranged to be supplied to the CPU 22. Further difference is that the CPU 22 is adapted to compare data of trigger temperature stored in advance in a ROM 22a or the like with the temperature data of the probe 9 detected by the temperature detection circuit 84. In case the temperature of the probe 9 becomes equal to or more that of the trigger temperature, the CPU 22 outputs a waveform pattern data PD, for starting output of an amplitude-modulated drive current described above.

With this configuration, the CPU 22 supplies a drive current of 100% output when a pedal of the foot switch 3 is stopped on. When the temperature of the probe 9 thereafter becomes equal to or more than a predetermined temperature, i.e. the trigger temperature, of 180 degrees in centigrade, for example, the CPU 22 outputs the waveform pattern data PD as described above to the D/A converter 28, so that an amplitude-modulated current signal is supplied to the handpiece 2.

Accordingly, a drive current of 100% output comes to be supplied until the temperature of the treatment device 5 of the handpiece 2 becomes equal to or more than a predetermined temperature.

### (Modification of the Sixth Embodiment)

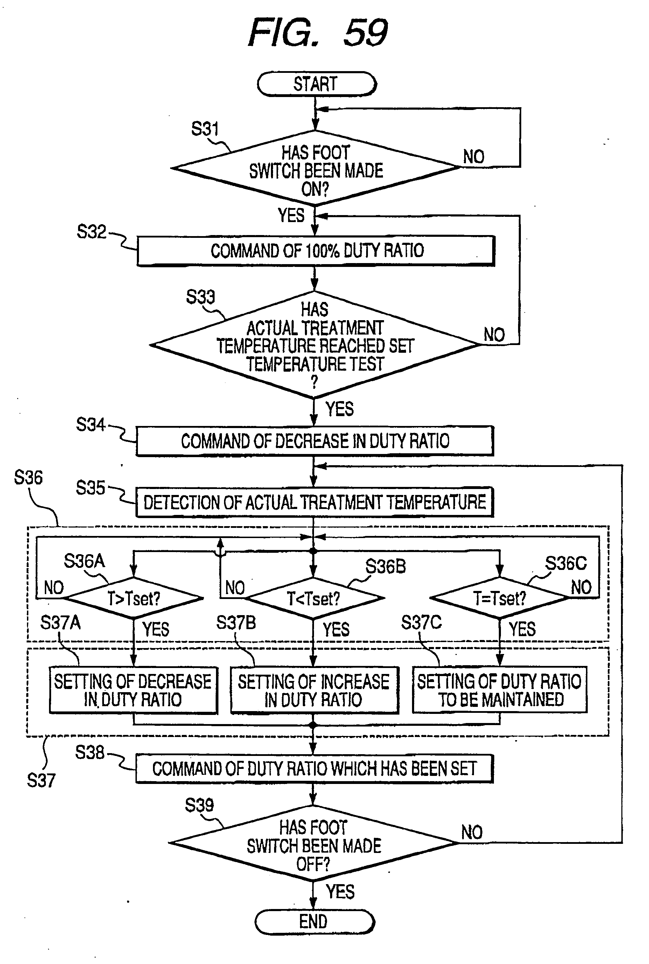

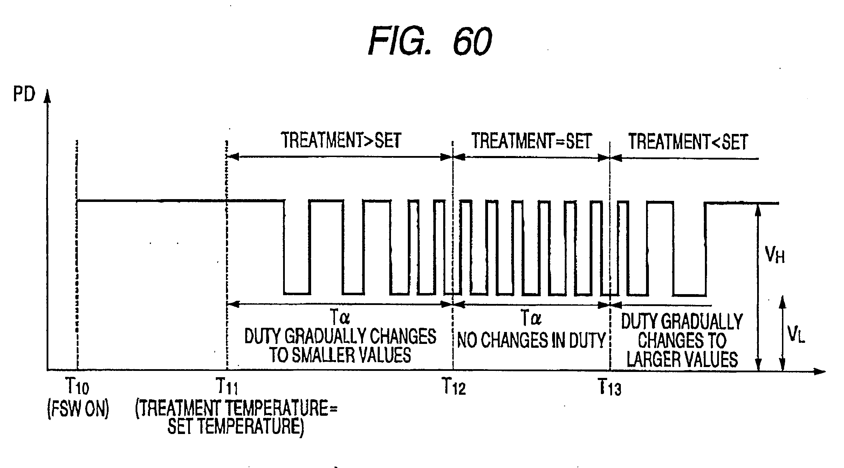

A modification is described with reference to Figs. 58 to 60.
The above sixth embodiment can be implemented by making a modification thereto as follows. In the sixth embodiment, heat generation caused at a treatment device by the frictional heat resulting from ultrasonic vibration of the probe 9, has been controlled based on an ideal temperature curve. Alternatively, instead of setting this ideal temperature curve, an upper limit of generated heat temperature may be set as a target value, and then a duty ratio or amplitude of a waveform pattern data PD may be controlled so that generated heat temperature follows the target value. This amplitude corresponds to the amplitude of voltage inputted to the D/A converter 28.
One example of this control is described in detail hereunder with reference to Figs. 58 to 60. As shown in Fig. 58, the temperature of a treatment device is gradually increased with time so as to achieve saturation at a specific temperature Tu, e.g. at 150 degrees in centigrade. Specifically, the upper limit Tu in the temperature curve is set as a target temperature for control, and actual temperature of the treatment device, which increases with the friction of the probe 9, is controlled so as to follow the target temperature, i.e. the upper limit (e.g. 150 degrees in centigrade). This control is performed by permitting the CPU 22 shown in Fig. 37 to change a duty ratio of a waveform pattern data PD (see Fig. 60) which corresponds to the voltage inputted to the D/A converter 28.
More specifically, the CPU 22 stands by while determining as to whether or not the foot switch 3 is on (step S31). When the foot switch 3 is determined to be on (time T₁₀ in Fig. 60), commands to issue a waveform pattern data PD of 100% duty ratio (step S32). Thereafter, the CPU 22 monitors a detection signal of the temperature detection circuit 84 to determine whether or not the actual treatment temperature has reached a set temperature Tset (i.e. target temperature of 150 degrees in centigrade, for example) (step S33). As a result of this determination, the waveform pattern data PD of 100% duty ratio (i.e., duty ratio = 100%) is maintained until the treatment temperature reaches the set temperature (NO at step S33).
On the other hand, when the treatment temperature becomes equal to the set temperature Tset (YES at step S33, and time T₁₁ in Fig. 60), a command is given to decrease the duty ratio at a predetermined rate for a specific period Tα (e.g., for several seconds) (step S34). As a result, as shown in Fig. 60, the duty ratio of the waveform pattern data PD is gradually decreased with time from the previous 100% output.
Then, the CPU 22 again monitors a detection signal from the temperature detection circuit 84 to compare actual treatment temperature with a set temperature (target temperature) (steps S35 and S36). In particular, the CPU 22 determines whether the treatment temperature is larger than the set temperature (step S36A), whether the treatment temperature is less than the set temperature (step S36B), and whether the temperature is equal to the set temperature (step S36C). Depending on the result of this determination, a command is issued to change or maintain a duty ratio (step S37). Particularly, when the treatment temperature is larger than the set temperature, the CPU 22 sets a duty ratio which decreases at a specific rate for the specific period Tα (step S37A). When the treatment temperature is less than the set temperature, the CPU 22 sets a duty ratio which increases at a specific rate for the specific period Tα (step S37B). When the treatment temperature is equal to the set temperature, the CPU 22 sets a duty ratio which maintains the ratio at the time for the specific period Tα (step S37C). The thus set duty ratio is outputted (step S38). The CPU 22 thereafter repeats the processes of steps S35 to S38 described above until a determination to turn off the foot switch 3 is made (step S39).
Thus, a duty ratio of a waveform pattern PD is changed as shown in Fig. 60, for example. Specifically, from the time T₁₁ when the treatment temperature has become equal to the set temperature, the duty ratio is decreased for the specific period Tα. Then, at the expiry of every specific period Tα from the time T₁₁, the treatment temperature is checked, and according to the result of the check, a command is issued (at time T₁₂, T₁₃, etc.) to maintain, increase or decrease the duty ratio. As a result, from when the foot switch 3 is stepped on, treatment temperature is promptly raised up to a set temperature (target temperature of 150 degrees in centigrade in this case) at a duty ratio of 100%, as shown in Fig. 58. At the time (time T11) when treatment temperature has reached a set temperature, control proceeds to the change of the duty ratio as described above. Thus, the duty ratio is controlled so that treatment temperature is approximately maintained at a set temperature.
In this way, temperature of a treatment device can be maintained at a desired value with the relatively simple control, i.e. to start control of a duty ratio when the temperature of the treatment device has reached a set temperature. This simple duty ratio control owes to a unique principle of an ultrasonic surgical apparatus, i.e. to perform incision and coagulation by using the frictional heat of the probe 9. This device is different from a surgical instrument, such as an electric cautery, in which treatment temperature drastically increases. In case of an ultrasonic surgical apparatus, its simplicity in duty ratio control owes to the smallness of a time constant of temperature transfer, and the readiness that a duty ratio, whether it is small or large, can be reflected, as it is, to treatment temperature. Such control of a duty ratio allows treatment temperature to be maintained around a set temperature. It should be understood that the temperature curve in Fig. 58 shows an ideal state, and thus practically, treatment temperature fluctuates within a predetermined tolerable width centered on a set temperature, due to the duty ratio control described above.
It should also be understood that in the treatment temperature control described above, temperature can be set at any value, and that an appropriate value within a range, for example, of 100 to 150 degrees in centigrade may be set to attain sufficient coagulation. As described above, as an alternative to a duty ratio of a waveform pattern data PD, its amplitude (voltage) may be controlled. In particular, by changing amplitude (V_{H}, V_{L}) shown in Fig. 60 according to actual treatment temperature, the temperature of a treatment device caused by friction can be controlled.
Additionally, the timing for transferring control to the change of duty ratio or amplitude, should not necessarily coincide with the time when treatment temperature becomes equal to a set temperature. For example, the transfer of control to the change of duty ratio or amplitude may be performed at the time when a formula expressed by "treatment temperature = set temperature - predetermined value β" is satisfied. This predetermined value □ is provided in view of the time constant of heat transfer of a treatment device described above. By this value β, the transfer of control to the change of duty ratio or amplitude can be performed a little earlier, so that overshooting of treatment temperature with respect to a set temperature can be surely suppressed. This predetermined value β, for example, may be only a few degrees in centigrade.
Instead of controlling a duty ratio or amplitude at the time when treatment temperature has become equal to a set temperature as described above, the ideal temperature profile shown in Fig. 58 may be stored in a memory in advance to control a duty ratio or amplitude along this temperature profile at the time when the foot switch 3 has been stepped on. This may allow control of treatment temperature with high accuracy.

### (Seventh Embodiment)

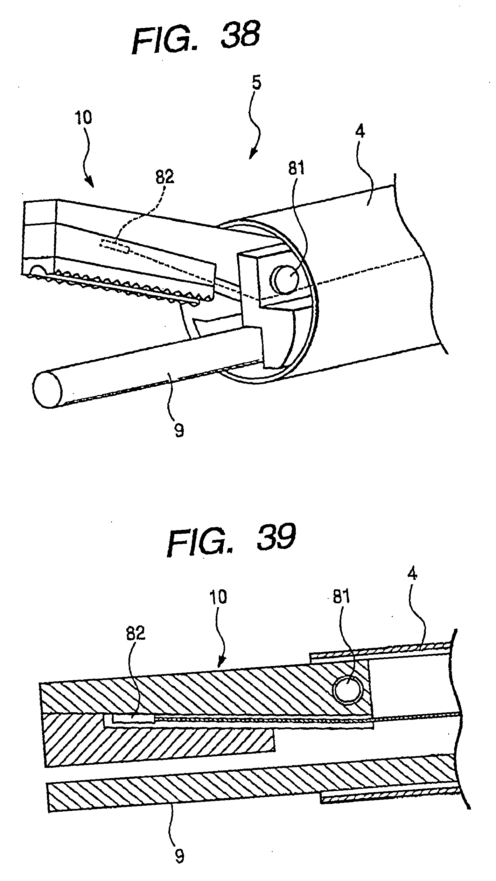

With reference to Figs. 38 and 39, a seventh embodiment of the ultrasonic surgical apparatus according to the present invention is described hereunder.
A temperature sensor 82 may be provided in a gripper 10 rather than in a probe 9. Fig. 38 is a perspective illustration of a treatment device 5 incorporating the temperature sensor 82. Fig. 39 is a cross section of a tip of the probe 9 shown in Fig. 38. In this case as well, the temperature sensor 82 can detect the temperature of the treatment device 5. Accordingly, an amplitude-modulated drive current is supplied to a handpiece 2 through the same circuit as shown in Fig. 37 when the temperature of the treatment device 5 becomes not lower than a predetermined trigger temperature.

In the example provided above, a drive current of constant amplitude has been outputted until a trigger signal is generated, and upon generation of a trigger signal, a predetermined amplitude-modulated drive current has been outputted. Alternatively, a first amplitude-modulated drive current may be outputted until a trigger signal is generated, and upon generation of a trigger signal, a second amplitude-modulated drive current, which is different from the first amplitude-modulated drive current, may be outputted.

### (Eighth Embodiment)

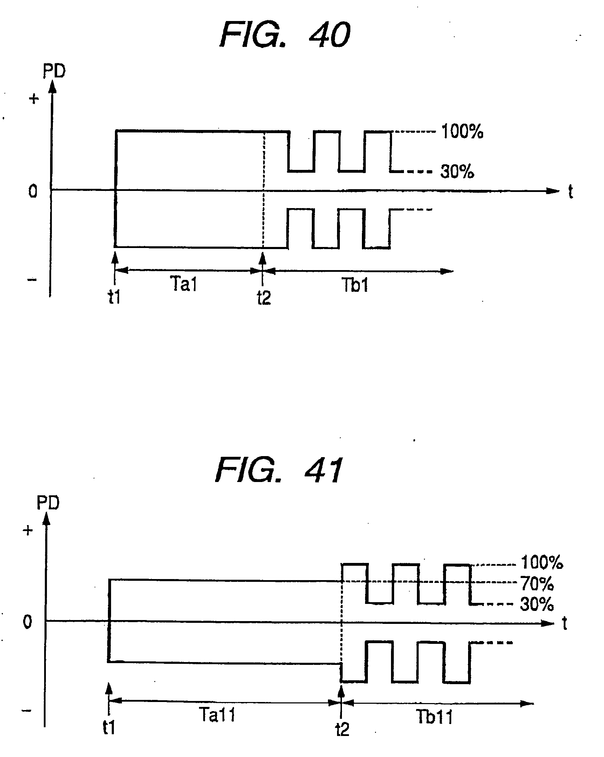

With reference to Fig. 40, an eighth embodiment of the ultrasonic surgical apparatus according to the present invention is described.
A time-out signal may be used as a predetermined trigger signal. Fig. 40 shows variation of a waveform pattern (fifteenth waveform pattern) in case a time-out signal serves as a trigger signal. For example, as shown in Fig. 40, after a foot switch 3 has been stepped on at time t1, a digital signal corresponding to a drive current of 100% output is transmitted to a D/A converter 28 from a CPU 22, so that the drive current of 100% output can be supplied to a handpiece 2. After expiry of a set period Ta1, a time-out signal is outputted from a timer at time t2. In a period Tb1 following the output of the time-out signal, a waveform pattern data PD is outputted to the D/A converter 28 from the CPU 22, so that a set amplitude-modulated drive current is supplied to the handpiece 2. In this case, a drive current which changes with 100% amplitude and 30% amplitude is supplied.
Is should be understood that the period Ta1, i.e. a period from time t1 to t2, may be set according to a value of a drive current which is outputted when the foot switch 3 is stepped on. In the case shown in Fig. 40, if a drive current after switching-on of the foot switch 3 at time t1 is of 70% output, the period Ta1 is set longer than the case of 100% output.

### (Ninth Embodiment)

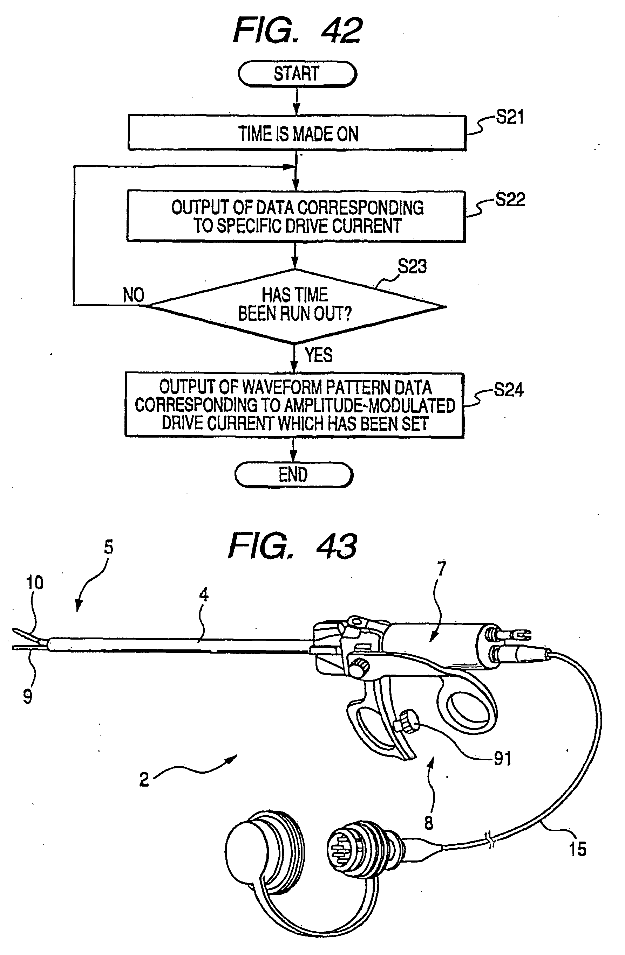

With reference to Figs. 41 and 42, a ninth embodiment of the ultrasonic surgical apparatus according to the invention is described.
Fig. 41 shows an example of variation of a waveform pattern (sixteenth waveform pattern) in which a time-out signal serves as a trigger signal. As shown in Fig. 41, an arrangement may be made such that, during a set period Ta11, a specific data corresponding to a drive current of 70% output is outputted to a D/A converter 28 from a CPU 22, so that a drive current of 70% output, for example, not 100%, is supplied, and that, during a period Tb11 following the output of the time-out signal, a waveform pattern data PD is outputted from the CPU 22 to the D/A converter 28, so that a set amplitude-modulated drive current can be supplied to a handpiece 2.

Fig. 42 is a flow diagram showing an example of a process flow of the CPU 22, which is performed so that a drive current, whose amplitude has been modulated according to a trigger signal, is supplied to the handpiece 2. The processes shown in Fig. 42 are executed when a pedal of the foot switch 3 is stepped on.
When a pedal of the foot switch 3 is stepped on, a timer for counting the predetermined period Ta1 (or Ta11) is turned on, or started up (step S21). This timer may be a software timer counted by the CPU 22, or a hardware timer. Subsequently, a specific drive current, e.g., a digital data corresponding to the 100% drive current in Fig. 39 or the 70% drive current in Fig. 41, is outputted (step S22) to the D/A converter 28 from the CPU 22.

A determination is the made (step S23) as to whether or not the time set at the timer has run out. If not, control returns to step S22. If the time has run out, the determination at step S23 results in YES. The CPU 22 then outputs (step S24) a waveform pattern data PD corresponding to a set amplitude-modulated drive current to the D/A converter 28 from the CPU 22.
As shown in Figs. 40 and 41, with the above arrangement, it is possible use a time-out signal as a predetermined trigger signal.

### (Tenth Embodiment)

With reference to Figs. 43 to 45, a tenth embodiment of the ultrasonic surgical apparatus according to the present invention is described.
An output signal of an output switch provided at a handpiece 2 may be utilized as a predetermined trigger signal.
Fig. 43 is a perspective illustration of the hand piece 2 in which an output switch is provided at one piece of an operation handle 8. When the operation handle 8 is gripped for closing, the one piece of handle comes close to the other piece of handle. An output switch 91 is provided at a face of the one piece of the operation handle 8, which is to be in contact with the other piece. When an operator operates the operation handle 8 for closing so that the output switch 91 is turned on, an output signal of the output switch 91 is supplied to the CPU 22 as a trigger signal.

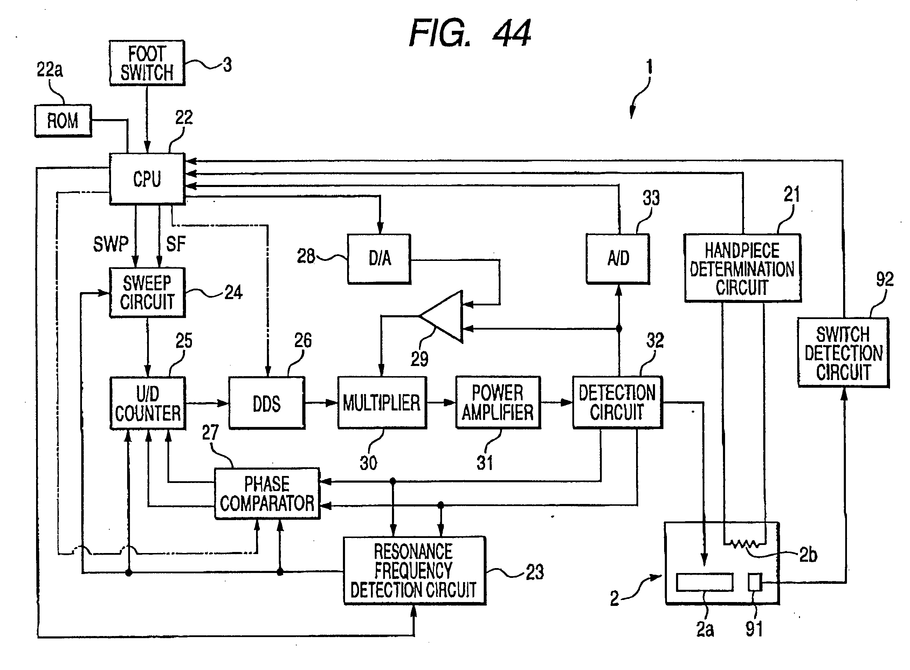

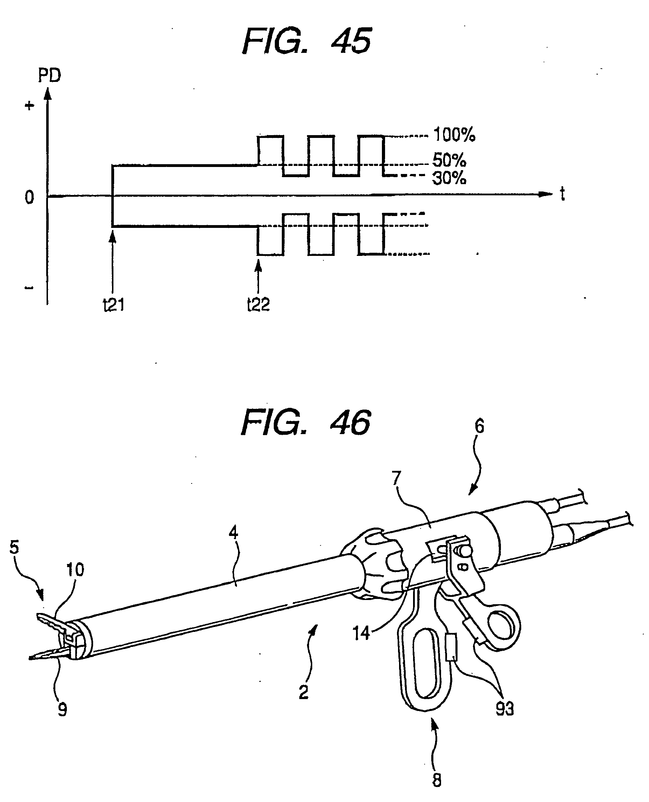

Fig. 44 is a block diagram showing a circuit configuration of a main unit 1 provided with a switch detection circuit 92 for receiving a signal from the output switch 91. Fig. 45 shows its effects. In Fig. 44, the same components as in the configuration shown in Fig. 2 are referred to by the same reference numerals, and description therefor is omitted.
A difference from the configuration shown in Fig. 2 is that, in the present embodiment, the switch detection circuit 92 is provided in the main unit 1, so that an on-signal indicative of the switching on of the output switch 91 detected by the switch detection circuit 92, is supplied to the CPU 22. Another difference is that, in the present embodiment, upon reception of the on-signal, the CPU 22 effects amplitude modulation described above to a drive current.

In this arrangement, when a pedal of the foot switch 3 is stepped on at time t21, the CPU 22 outputs a 50% drive current, for example, for an initial period after time t21. Thereafter, when the output switch 91 is turned on at time t22, the CPU 22 outputs the above waveform pattern to the D/A converter 28, so that an amplitude-modulated drive signal may be supplied to the handpiece 2.
Accordingly, an amplitude-modulated drive current is outputted to the handpiece 2 only when an operator uses the handpiece 2 to hold living tissue.

### (Eleventh Embodiment)

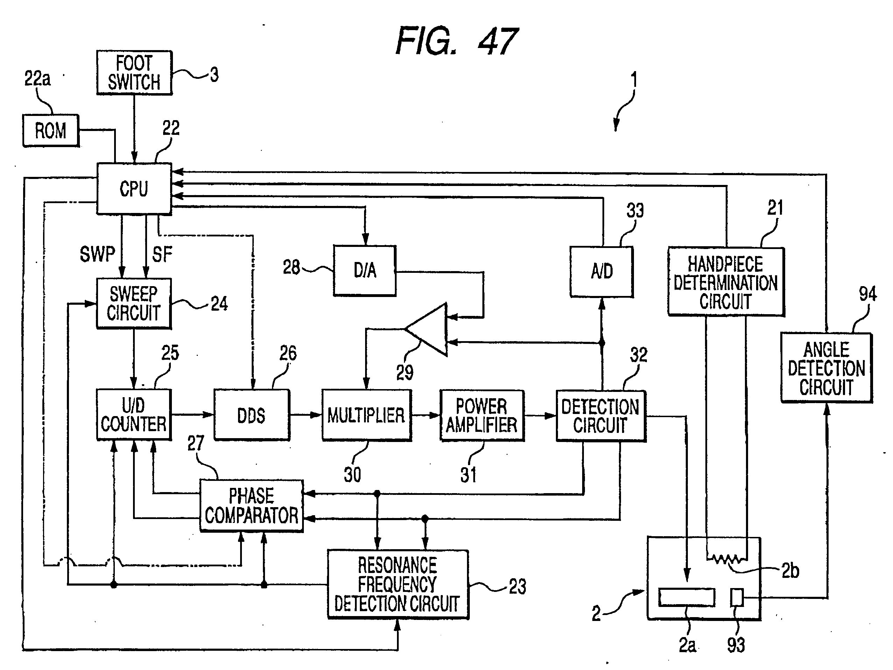

With reference to Fig. 46 and 47, an eleventh embodiment of the ultrasonic surgical apparatus according to the present invention is described.
An output signal of an angle sensor provided at a handpiece 2 may be used as a predetermined trigger signal.
Fig. 46 is a perspective illustration of the handpiece 2 provided with an angle sensor 93 at the operation handle 8. The angle sensor 93 is constituted of a plurality of light receiving elements and a light emitting element. The plurality of linearly arranged light receiving elements are provided at one piece of the scissors-shaped operation handle 8, and the light emitting element is provided at the other piece. When the two pieces of the handle are gripped and operated so as to be close to each other, the one piece of the handle pivotally moves about a pivotal center while the other piece remains stationary. Thus, among the plurality of light receiving elements, those which currently receive light from the light emitting element are successively switched to others because an incidence angle of the light emitted from the light emitting element changes with the pivotal movement of the one piece of the handle. In this way, the light receiving elements, which currently receive light, are allowed to successively change according to an angle made by the two pieces of the operation handle 8 operated by an operator, or according to the amount of closing movement of the two pieces. Accordingly, an angle detection circuit 94 is enabled to detect the angle made by the two pieces based on a detection signal from the plurality of light receiving elements.

Fig. 47 is a block diagram of a circuit configuration of a main unit 1 provided with the angle detection circuit 94 for receiving a signal from the angle sensor 93. The same components as in the configuration shown in Fig. 2 are referred to by the same reference numerals, and description therefor is omitted.
A difference from the configuration shown in Fig. 2 is that, in the present embodiment, the angle detection circuit 94 is provided to the main unit 1, so that a detection signal detected by the angle sensor 93 is transmitted to the CPU 22 as an angle signal. Another difference is that, in the present embodiment, upon reception of the angle signal, the CPU 22 compares the angle signal with a preset angle, and if the angle signal is equal to or less than the preset angle, effects amplitude modulation described above to a drive current serving as a drive signal.

In this arrangement, the timing when an angle made between the two pieces of the operation handle 8 becomes equal to or less than a preset angle, serves as a trigger signal. With this trigger signal, the CPU 22 outputs the waveform pattern data PD as described above to the D/A converter 28, so that an amplitude-modulated drive current is supplied to the handpiece 2.

In the above description, an example has been given in which linearly arranged light receiving elements are used. Alternatively, a single light receiving element may be given at a predetermined angle position to notify the CPU 22 of a presence of an output.

Thus, an amplitude-modulated drive current comes to be outputted to the handpiece 2 only when an operator uses the handpiece 2 to hold living tissue.

### (Twelfth Embodiment)

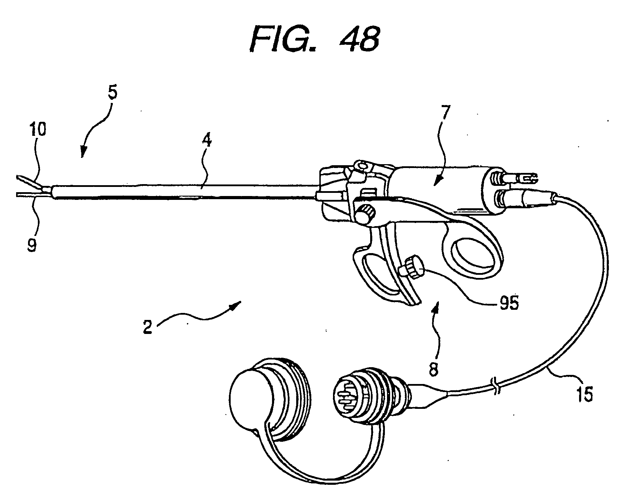

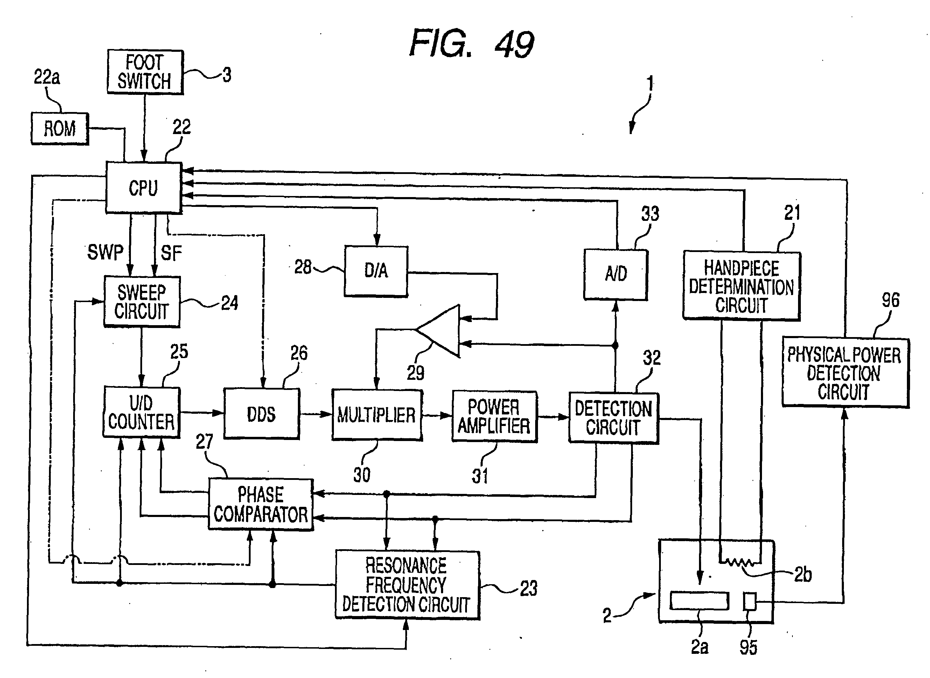

With reference to Figs. 48 and 49, a twelfth embodiment of the ultrasonic surgical apparatus according to the present invention is described.
An output signal of a power sensor provided in a handpiece 2 may be utilized as a predetermined trigger signal.
Fig. 48 is a perspective illustration of the handpiece 2 in which a physical power sensor 95 is provided at an operation handle 8. The physical power sensor 95 is a pressure sensor, for example. The physical power sensor 95 is provided at one of two pieces of the scissors-shaped operation handle 8. When the two pieces of the handle is gripped and operated so that they come close to each other, the one piece of the handle pivotally moves about a pivotal center while the other piece remains stationary, so that the other piece of the handle comes into contact with the physical power sensor 95. After the contact, when the operator operates the operation handle 8 with stronger physical power, the physical power sensor 95 outputs a signal corresponding to the physical power given by the operator to a physical power detection circuit 96. Thus, the physical power detection circuit 96 is enabled to detect the physical power.

Fig. 49 is a block diagram showing a circuit configuration of a main unit 1 provided with the physical power detection circuit 96 for receiving a signal from the physical power sensor 95. The same components as the configuration shown in Fig. 2 are referred to by the same reference numerals, and description therefor is omitted.
A difference from the configuration shown in Fig. 2 is that the present embodiment is provided with the physical power detection circuit 96 in the main unit 1, which receives a detection signal detected by the physical power sensor 95 to transmit a physical power signal, e.g. a pressure signal, to the CPU 22. Another difference is that, upon reception of the physical power signal, the CPU 22 compares the physical power signal with a preset value, and when the physical power signal becomes equal to or more than the preset value, amplitude modulation described above is effected to a drive current (drive signal).

In this arrangement, the timing when the operation handle 8 is operated by an operator to a physical power equal to or more than a predetermined value, serves as a trigger signal. With this trigger signal, the CPU outputs the waveform pattern data PD described above to the D/A converter 28, so that an amplitude-modulated drive current is supplied to the handpiece 2.

As a result, an amplitude-modulated drive current is outputted to the handpiece 2 only when an operator uses the handpiece 2 to hold living tissue with a predetermined physical power.

### (Thirteenth Embodiment)

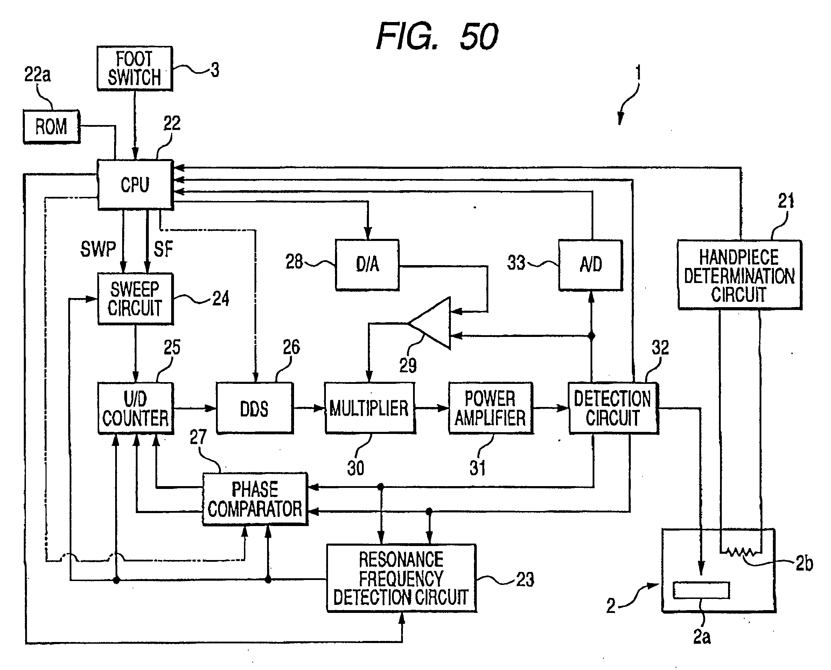

With reference to Fig. 50, a thirteenth embodiment of the ultrasonic surgical apparatus according to the present invention is described.
Impedance of a handpiece 2 may be used as a predetermined trigger signal.
In incision treatment, for example, a living tissue portion is held between a probe 9 and a gripper 10. Since impedance of the handpiece 2 changes by holding living tissue, the change of the impedance can be used as a trigger signal.

Fig. 50 is a block diagram showing a circuit configuration of a main unit 1 which utilizes impedance as a trigger signal. The same components as in the configuration shown in Fig. 2 are referred to by the same reference numerals, and description therefor is omitted.
A difference from the configuration shown in Fig. 2 is that the present embodiment is so configured that the detection circuit 32 is provided with an impedance detection function so that a detected impedance signal is supplied to the CPU 22. Another difference is that, in the present embodiment, upon reception of a detected impedance signal, the CPU 22 compares the impedance signal with a preset value, and when the impedance signal becomes equal to or more than the preset value, effects amplitude modification described above to an outputted drive current.

In this arrangement, the timing when the operation handle 8 is operated by an operator for a predetermined operation that changes impedance, serves as a trigger signal. With this trigger signal, i.e. with the change of impedance, the CPU 22 outputs the waveform pattern data PD described above to the D/A converter 28, so that an amplitude-modulated drive current is supplied to the handpiece 2.

### (Fourteenth Embodiment)

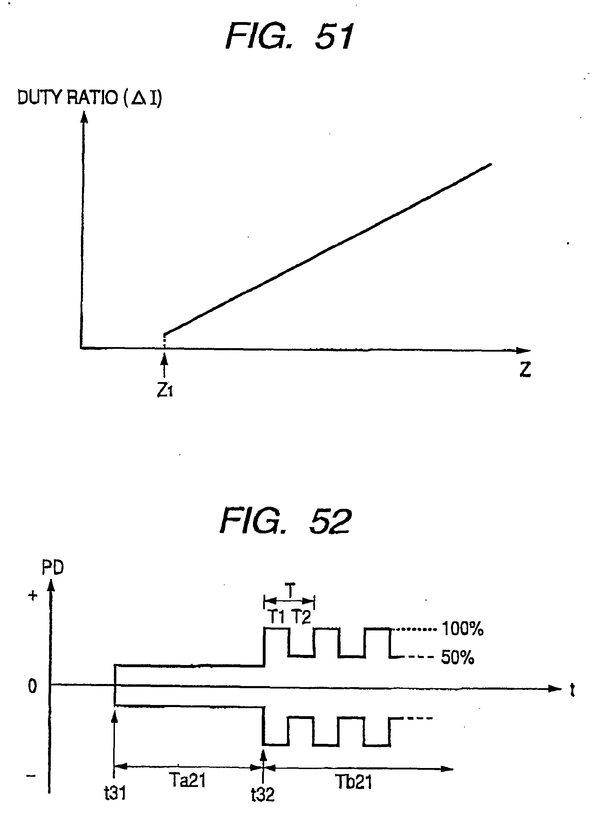

With reference to Fig. 51 to 53, a fourteenth embodiment of the ultrasonic surgical apparatus according to the present invention is described.
An arrangement may be so made that a preset value of a drive current, i.e. a duty ratio, is changed according to detected impedance.
Fig. 51 is a graph showing the change of a duty ratio relative to the change of impedance. As can be seen from Fig. 51, no amplitude modulation is effected until impedance reaches a preset value Z1. However, when impedance becomes equal to or more than the preset value Z1, the impedance is permitted to be associated with a duty ratio, so that a duty ratio in amplitude modification, i.e. T1/T or T1/T2 in this case, is increased as impedance increases. The association of impedance with a duty ratio may be stored in advance in a ROM or the like as a table data for the CPU 22 to refer to the table data, or may be obtained through an operation of the CPU 22 based on a predetermined formula. Based on detected impedance, the CPU 22 reads out or calculates a duty ratio with reference to the relation between impedance and a duty ratio shown in Fig. 51. The CPU 22 then outputs a waveform pattern data PD corresponding to an obtained duty ratio to the D/A converter 28.

Fig. 52 shows an example of a waveform pattern (eighteenth waveform pattern) outputted from the CPU 22. When the foot witch 3 is depressed at time t31, a data of a preset specific output (in this case 30%, not 100%) is outputted to the D/A converter 28 from the CPU 22. Then, when impedance becomes equal to or more than the preset value Z1 at time t32, the CPU 22 outputs a waveform pattern data PD of a duty ratio that has been set according to Fig. 51 to the D/A converter 28, so that an amplitude-modulated drive current is supplied to the handpiece 2. During a period Ta21, i.e. from time t31 to time t32, a specific data without amplitude modulation is outputted. During a period Tb21, i.e. after time t32, a waveform pattern data PD of a duty ratio corresponding to detected impedance is outputted to the D/A converter 28 from the CPU 22 based on the data shown in Fig. 51.

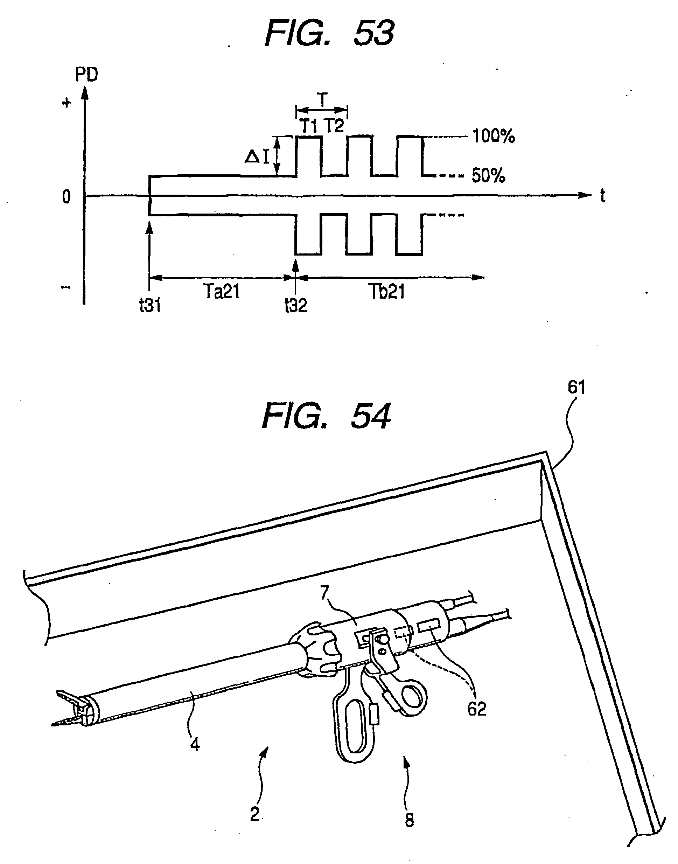

As a modification, amplitude modulation may be carried out as shown in Fig. 53. Fig. 53 shows an example of a waveform pattern data PD (nineteenth waveform pattern data) outputted from the CPU 22. When the foot switch 3 is depressed at time t31, a data of a preset specific output (in this case 50%, not 100%) is outputted to the D/A converter 28 from the CPU 22. Then, when impedance becomes equal to or more than the preset value Z1 at time t32, the CPU 22 outputs a waveform pattern data PD to the D/A converter 28. In this regard, the waveform pattern data PD is the one that has been modified by an amplitude increase of ΔI of a drive current according to detected impedance, with a duty ratio being kept as predetermined. In Fig. 53, the vertical axis of Fig. 51 is reflected as an increase ΔI.

In this way, an amplitude-modulated drive current is outputted to the handpiece 2 only when the handpiece 2 is used by an operator to hold living tissue.

As described above, the CPU 22 outputs a waveform pattern data to the D/A converter 28, so that an amplitude-modulated drive current is supplied to the handpiece 2 only when an operator uses the handpiece 2 for incision treatment or the like. In the examples provided above, impedance, an output switch, an angle, physical power and the like have been introduced as a trigger signal, however, other alternatives may be used as a trigger signal.

### (Fifteenth Embodiment)

With reference to Fig. 54, a fifteenth embodiment of the ultrasonic surgical apparatus according to the present invention is described.
An RF-ID tag may be stuck onto a handpiece 2 with information on the handpiece 2 being recorded on the RF-ID tag. In this case, an arrangement may be so made that an operator and/or nurses can recognize that a handpiece 2 carries an RF-ID tag when the handpiece 2 is placed in a tray or the like.
For this purpose, as shown in Fig. 54, an RF-ID tag 62 may be provided at a certain position on a surface of the handpiece 2, so that an operator and/or nurses can see the tag when they see the handpiece 2 placed in a tray 61.

If an operator and/or nurses can recognize that the handpiece 2 is provided with the RF-ID tag 62, they can bring the RF-ID tag 62 close to a reader to transmit information stored therein, such as a waveform pattern data, to the CPU 22 in the main unit 1, so that the main unit 1 can output a waveform pattern data suitable for the handpiece 2.

In order for an operator and/or nurses to recognize that the handpiece 2 carries the RF-ID tag 62, an arrangement may be so made that the FR-ID tag 62 is provided at a position that can be seen when the handpiece 2 is placed in the tray 61 with whichever side thereof being turned up. For example, as shown by a dotted line in Fig. 54, the RF-ID tag 62 may be provided not only on one side of a case 7 but also on the other side of the case 7 so as to be recognized when placed in a tray or the like with whichever side being turned up.

Similar to the foregoing embodiments, according to the fifteenth embodiment, use of the waveform pattern data of a drive current as described above may realize an ultrasonic surgical apparatus which does not allow deterioration of incision capability, while suppressing heat generation of a treatment device.

### (Sixteenth Embodiment)

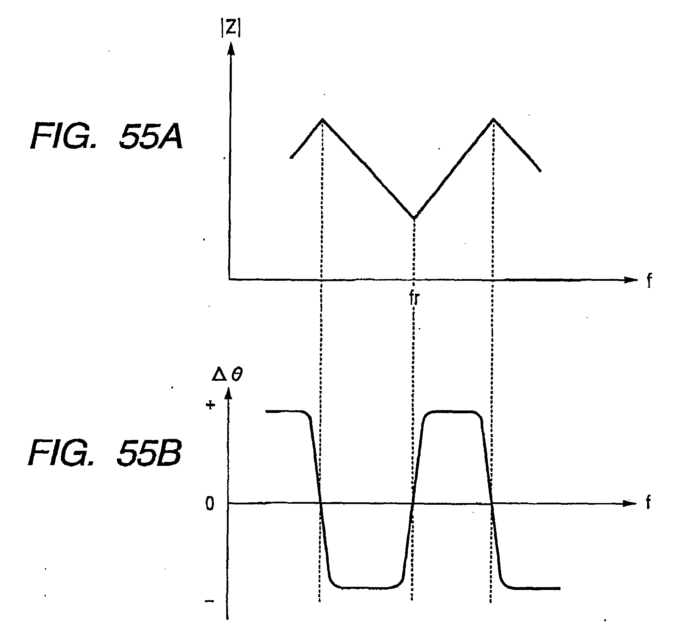

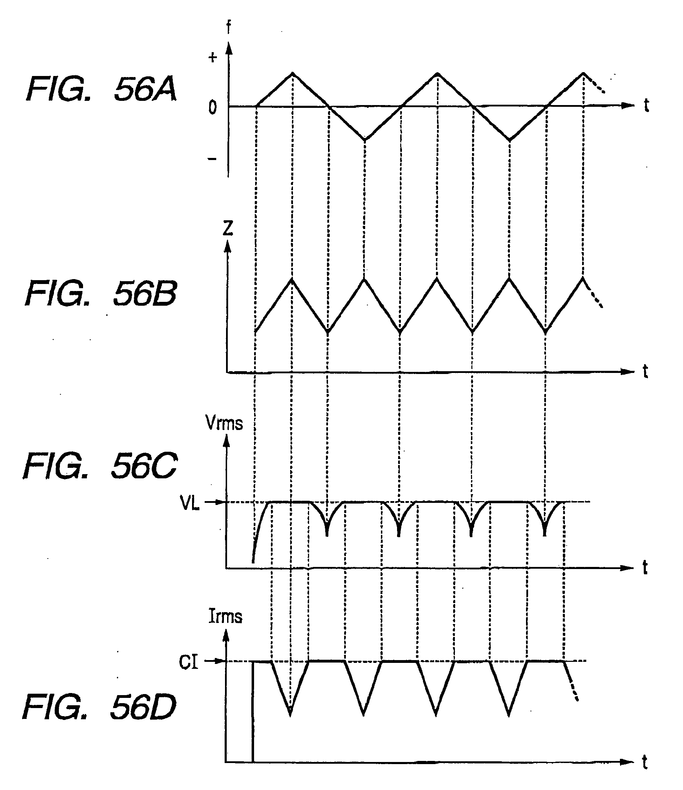

With reference to Figs. 55A, 55B and 56A-56D, a sixteenth embodiment of the ultrasonic surgical apparatus according to the present invention is described.
In order to suppress heat generation of a treatment device, the ultrasonic surgical apparatus according to the sixteenth embodiment utilizes frequency modulation for the modulation of a drive current.
An electric circuit configuration of the ultrasonic surgical apparatus according to the present invention is substantially the same as the electric circuit configuration shown in Fig. 2. However, as shown by a dash-dot-dot line in Fig. 2, there is provided a signal line for outputting a control signal to the phase comparator 27 from the CPU 22, or a signal line for outputting a control signal to the DDS 26 from the CPU 22. Further, a voltage limiter is provided for the function of constant-current supply in the ultrasonic surgical apparatus. In other words, the ultrasonic surgical apparatus is adapted not to apply any voltage equal to or more than a preset value onto the handpiece 2.

Figs. 55A and 55B show characteristics of impedance and phase difference, respectively, centered on a resonance frequency fr of an ultrasonic transducer 2a. As shown in Fig. 55A, the impedance Z becomes the smallest at the resonance frequency fr where the phase difference between voltage and current is zero, thereby achieving good energy efficiency. Thus, in the first embodiment described above, the resonance frequency fr is detected, and a frequency "f" of a supplied drive current is locked on the resonance frequency, as shown in Figs. 55A and 55B. As shown in Fig. 55B, at the resonance frequency fr, a phase difference Δθ between voltage and current is zero.

Figs. 56A to 56D each show a waveform diagram for explaining the processes performed by the CPU 22 in the present embodiment. After detecting the resonance frequency fr, the CPU 22 changes the frequency f of a drive current to be supplied to the ultrasonic transducer 2a centering on the resonance frequency fr. In the present embodiment, as shown in Fig. 56A, the CPU 22 either supplies a phase offset amount to the phase comparator 27, or supplies a frequency offset amount to the DDS 26, so that the frequency f of a drive current supplied to the handpiece 2 repeats periodical increase and decrease centering on the frequency fr.

The phase comparator 27 supplies, in the first place, an up signal or a down signal to the U/D counter 25 so that a phase difference turns to zero, while the CPU 22 changes a value of the up signal or the down signal to be supplied, so that the frequency of a drive current is offset as described above. Further, the DDS 26 outputs a frequency signal based on a value set in the U/D counter 25, while the CPU 22 changes the value set in the U/D counter 25 so that the frequency of a drive current is offset as described above. In particular, the CPU 22 is capable of controlling the frequency of a drive current to periodically repeat increase and decrease centering on the center frequency fr. In this way, in the impedance and phase characteristics shown in Figs. 55A and 55B, the CPU 22 controls the phase comparator 27 or the DDS 26 so that the frequency f is periodically offset from the resonance frequency fr.

The transducer 2a vibrates with the best energy efficiency when a drive current having the resonance frequency fr is supplied to the handpiece 2. However, when the handpiece 2 is supplied with a drive current whose frequency f varies with respect to the center frequency, repeating increase and decrease as shown in Fig. 56A, the transducer 2a vibrates with bad energy efficiency, i.e. with varying energy efficiency.

As shown in Fig. 56A, the CPU 22 supplies a control signal to the phase comparator 27 or the DDS 26, so that a jagged drive current having an offset frequency f with respect to the center frequency fr is supplied to the handpiece 2. As shown in Fig. 56B, the impedance Z of the handpiece 2 varies as the frequency f varies. The impedance Z is the smallest at the resonance frequency fr.

As shown in Fig. 56C, an effective value of output voltage (Vrms) is prevented from being equal to or more than a limiting value VL by the voltage limiter. As shown in Fig. 56D, since the impedance Z increases with limited output voltage, the effective value of a drive current (Irms) decreases lower than a constant current CI according to the impedance Z.

Thus, according to the sixteenth embodiment, the frequency f can be periodically varied within a range including the resonance frequency fr. In this way, an ultrasonic surgical apparatus which does not reduce the incision capability but suppresses heat generation of a treatment device can be achieved.

It should be understood that, in the sixteenth embodiment as well, an arrangement may be so made that frequency modulation is performed in response to a preset trigger signal as described in the foregoing embodiments. For example, frequency of a drive current may be changed by using a trigger signal, such as an output from a temperature sensor and a time-out signal from a timer as described referring to Figs. 34 to 53.

It should also be understood that frequency modulation may be performed in accordance with the type of a handpiece.

to [0139] Various embodiments and modifications have been described with respect to the ultrasonic surgical apparatus and the method for driving the ultrasonic surgical apparatus according to the present invention. Such devices and methods according the present invention are not necessarily limited to the ones described herein, but may include those which can be implemented with further modification without departing from the spirit of the present invention.

## Claims

1. An ultrasonic surgical apparatus comprising:
an ultrasonic transducer generating vibration in response to a drive signal to be given;
a treatment device having a probe to which the vibration is transferred from the ultrasonic transducer to effect treatment with the vibration;
a signal generator generating an AC (alternating current) signal for driving the ultrasonic transducer; and
a modulator for modulating the AC signal generated by the signal generator to produce the drive signal to be given to the ultrasonic transducer.

2. The apparatus of claim 1, comprising a gripper gripping a portion to be treated of an object in cooperation of the probe.

3. The apparatus of claim 2, wherein the modulator is configured to modulate the AC signal so that at least one of an amplitude and a duty ratio of the drive signal is changed.

4. The apparatus of claim 3, wherein the modulator is configured to change the amplitude of the AC signal based on a predetermined waveform pattern.

5. The apparatus of claim 3, wherein the waveform pattern is a plurality of continuous pulse waveforms each having a predetermined duty ratio.

6. The apparatus of claim 5, wherein, assuming that each of the pulse waveforms has a period of time T1 in which the amplitude is high and a period of time T2 in which the amplitude is low, each of the pulse waveforms has a duty ratio, which is defined as "T1/(T1+T2)", of 5 to 10 percents and has a cycle, which is defined as "T1+T2", of 0.1 to 1 seconds.

7. The apparatus of claim 6, wherein the duty ratio "T1/(T1+T2)" is 5 to 50 percents and the cycle "T1+T2" is 0.4 to 1 seconds.

8. The apparatus of claim 5, wherein the modulator is configured to change at least one of the duty ratio and the amplitude in response to a triggering signal.

9. The apparatus of claim 5, wherein the modulator comprises change means changing the duty ratio and the amplitude in response to a trigger to be given during the plurality of continuous waveforms are issued.

10. The apparatus of claim 1, wherein the modulator comprises modulating means starting the modulation of the drive signal in response to a trigger to be given.

11. The apparatus of claim 10, wherein the trigger is a timing signal to be produced when the treatment device is used.

12. The apparatus of claim 11, wherein the timing signal is any one selected from a group of signals composed of a timing signal produced at a timing when the probe presents a predetermined temperature, a timing signal produced at a timing when a predetermined timer shows a time-out state, a timing signal produced at a timing when the treatment device is gripped to be subjected to a predetermined treatment operation, and a timing signal produced at a timing when the treatment device presents a predetermined impedance value.

13. The apparatus of claim 1, wherein the modulator is configured to produce the drive signal by changing a frequency of the AC signal.

14. The apparatus of claim 1, comprising a determination unit automatically determining a type of the treatment device,
wherein the modulator is configured to produce the drive signal by performing the modulation on the AC signal, the modulation being depending on a type of the treatment device.

15. The apparatus of claim 1, comprising a temperature detector detecting a temperature at a treated portion of an object to be treated by the treatment device,
wherein the modulator comprises means for controlling a modulated state of the AC signal so as to allow the temperature at the treated portion detected by the temperature detector to follow up a predetermined target temperature.

16. An ultrasonic surgical apparatus comprising:
an ultrasonic transducer generating vibration in response to a drive signal to be given;
a treatment device having a probe to which the vibration is transferred from the ultrasonic transducer to effect treatment with the vibration;
signal generating means for generating an AC (alternating current) signal for driving the ultrasonic transducer; and
modulation means for modulating the AC signal generated by the signal generator to produce the drive signal to be given to the ultrasonic transducer.

17. A method of driving a treatment device with an ultrasonic transducer, comprising steps of:
generating an AC (alternating current) signal;
producing a drive signal by modulating an amplitude of the AC current generated, the modulation being such that, assuming that the drive signal has a period of time T1 in which the amplitude is high and a period of time T2 in which the amplitude is low, the drive signal has a duty ratio, which is defined as "T1/ (T1 +T2)", of 5 to 10 percents and has a cycle, which is defined as "T1+T2", of 0.1 to 1 seconds; and
supplying the drive signal to the ultrasonic transducer.

18. The method of claim 17, wherein the duty ratio "T1 /(T1+T2)" is 5 to 50 percents and the cycle "T1+T2" is 0.4 to 1 seconds.

19. The method of claim 17, further comprising steps of automatically determining a type of the treatment device,
wherein the modulation step is configured to produced the drive signal by performing the modulation on the AC signal, the modulation being depending on a type of the treatment device.

20. A treatment method using an ultrasonic treatment device, comprising steps of:
touching a probe coupled with an ultrasonic transducer to a portion to be treated of an object;
producing a drive signal by modulating an amplitude of an AC (alternating current) signal, the modulation being such that, assuming that the drive signal has a period of time T1 in which the amplitude is high and a period of time T2 in which the amplitude is low, the drive signal has a duty ratio, which is defined as "T1/(T1+T2)", of 5 to 10 percents and has a cycle, which is defined as "T1+T2", of 0.1 to 1 seconds; and
supplying the drive signal to the ultrasonic transducer to cause vibration generated by the ultrasonic transducer is transferred to the portion to be treated via the probe.
